# EUROPEAN PATENT SPECIFICATION

(11) **EP 3 251 692 B1**
(45) Date of publication and mention of the grant of the patent: **27.09.2023**
(21) Application number: 17180615.1
(22) Date of filing: 14.05.2012
(51) Int. Cl.: A61K 39/155

(54) **HENDRA AND NIPAH VIRUS G GLYCOPROTEIN IMMUNOGENIC COMPOSITIONS**
IMMUNOGENE HENDRA- UND NIPAH-VIRUS-G-GLYCOPROTEIN-ZUSAMMENSETZUNGEN
COMPOSITIONS IMMUNOGÈNES DE GLYCOPROTÉINE G DE VIRUS HENDRA ET NIPAH

(30) Priority: 13.05.2011 US 201161485992 P
(43) Date of publication of application: 06.12.2017
(62) Divisional of application: 12785009.7
(73) Proprietor: Zoetis Services LLC, Parsippany, NJ 07054 (US); HENRY M. JACKSON FOUNDATION FOR THE ADVANCEMENT OF MILITARY MEDICINE, INC., Bethesda, MD 20817 (US)
(72) Inventor: ELHAY, Martin, Yarraville, Victoria 3013 (AU); BRODER, Christopher C., Bethesda, MD 20817 (US); HUANG, Jin-An, Box Hill North, VIC 3129 (AU)
(74) Representative: D Young & Co LLP

(56) References cited:
- WO-A2-2006/115548
- US-A1- 2002 081 329
- US-A1- 2009 041 772
- B. A. MUNGALL ET AL: "Feline Model of Acute Nipah Virus Infection and Protection with a Soluble Glycoprotein-Based Subunit Vaccine", JOURNAL OF VIROLOGY, vol. 80, no. 24, 27 September 2006 (2006-09-27), pages 12293-12302, XP055151022, ISSN: 0022-538X, DOI: 10.1128/JVI.01619-06
- MCEACHERN J A ET AL: "A recombinant subunit vaccine formulation protects against lethal Nipah virus challenge in cats", VACCINE, ELSEVIER LTD, GB, vol. 26, no. 31, 23 July 2008 (2008-07-23) , pages 3842-3852, XP022822176, ISSN: 0264-410X, DOI: 10.1016/J.VACCINE.2008.05.016 [retrieved on 2008-06-02]

## Description

### Field of the Disclosure

The present disclosure relates to immunogenic and vaccine compositions comprising a G glycoprotein from Hendra virus (HeV) and/or Nipah virus (NiV) and to methods of use relating thereto.

### Description of the Background

Recurrent outbreaks of NiV resulting in significant numbers of human fatalities have recently been problematic (see e.g. Butler (2000) Nature 429, 7). HeV is also known to cause fatalities in human and animals and is genetically and immunologically closely related to NiV. There are presently no vaccines or therapeutics for prevention of infection or disease caused by Nipah virus or Hendra virus. Both Nipah virus and Hendra virus are United States, National Institute of Allergy and infectious Disease, category C priority agents of biodefense concern. Further, as these viruses are zoonotic Biological Safety Level-4 agents (8SL-4), production of vaccines and/or diagnostics, with safety is very costly and difficult. Thus, there is a need for Nipah virus or Hendra virus vaccines and diagnostics that allow for high throughput production of vaccines and/or diagnostics.

Paramyxoviruses such as HeV and NiV possess two major membrane-anchored glycoproteins in the envelope of the viral particle. One glycoprotein is required for virion attachment to receptors on host cells and is designated as either hemagglutinin-neuraminidase protein (HN) or hemagglutinin protein (H), and the other is glycoprotein (G), which has neither hemagglutination nor neuraminidase activities. The attachment glycoproteins are type II membrane proteins, where the molecule's amino (N) terminus is oriented toward the cytoplasm and the protein's carboxy (C) terminus is extracellular. The other major glycoprotein is the fusion (F) glycoprotein, which is a trimeric class I fusogenic envelope glycoprotein containing two heptad repeat (HR) regions and a hydrophobic fusion peptide. HeV and NiV infect cells though a pH-independent membrane fusion process into receptive host cells through the concerted action of their attachment G glycoprotein and F glycoprotein following receptor binding. The primary function of the HeV and NiV attachment G glycoprotein is to engage appropriate receptors on the surfaces of host cells, which for the majority of well-characterized paramyxoviruses are sialic acid moieties. The HeV and NiV G glycoproteins utilize the host cell protein receptors ephrin B2 and/or ephrin B3 and antibodies have been developed which block viral attachment by the G glycoprotein (WO2006137931, Bishop (2008) J. Virol. 82: 11398-11409). Further, vaccines have been developed which also use the G glycoprotein as a means for generating an immunoprotective response against HeV and NiV infection (WO2009117035).
Mungall et al (2006, J Virol, 80(24); 12293-12302) disclose a feline model of acute Nipah virus infection and protection with a soluble protein G-based subunit vaccine.

Dose-site reactivity is a major concern for both the veterinary and human use of Quil A in vaccine preparations. One way to avoid this toxicity of Quil A is the use of immunostimulating complexes (Rajput (2007) J. Zhejiang Univ. Sci. B, 8: 153-161). This is primarily because Quil A is less reactive when incorporated into immunostimulating complexes, because its association with cholesterol in the complex reduces its ability to extract cholesterol from cell membranes and hence its cell lytic effects. in addition, a lesser amount of Quil A is required to generate a similar level of adjuvant effect. The immunomodulatory properties of the Quil A saponins and the addition benefits to be derived from these saponins when they are incorporated into an immunostimulating complex have been described in WO2000041720.

The combination of HeV and/or NiV G glycoproteins with immunostimulating complexes in a single vaccine represents an advancement in developing effective HeV and NiV vaccines given the potential for enhanced immunoreactivity with decreased adjuvant side effects when these components are administered in combination.

### Summary of the Invention

The invention can be summarized by the following numbered paragraphs:
1. A vaccine comprising:
   a. a soluble fragment of the Nipah virus G glycoprotein, said fragment consisting of amino acids 71 to 602 of SEQ ID NO: 4 or a sequence at least 95% identical thereto;
   b. an immunostimulatory complex (ISC), said ISC comprising a saponin; a phospholipid selected from the group consisting of phosphatidyl choline (PC), dipalmitoyl phosphatidyl choline (DPPC), phosphatidic acid (phosphatidate) (PA), phosphatidylethanolamine (PE), phosphatidylserine (PS), phosphatidylinositol (PI), phosphatidylinositol phosphate (PIP), phosphatidylinositol bisphosphate (PIP2), phosphatidylinositol triphosphate (PIP3), phosphorylcholine (SPH), ceramide phosphorylethanolamine (Cer-PE) and ceramide phosphorylglycerol; and cholesterol; and
   c. one or more excipients;
   for use in prevention of an infection caused by Hendra and/or Nipah virus in a horse or a pig, wherein said vaccine is administered to said horse or said pig in a first dose and a second dose, wherein each of said doses comprises 50-100 µg of said soluble fragment of the Nipah virus G glycoprotein.
2. The vaccine for use according to paragraph 1, wherein each dose comprises 100 µg of said soluble fragment of the Nipah virus G glycoprotein.
3. The vaccine for use according to paragraph 1 or paragraph 2, wherein the soluble Nipah virus G glycoprotein is present in dimer form.
4. The vaccine for use according to paragraph 1 or paragraph 2, wherein the soluble Nipah virus G glycoprotein is present in tetramer form.
5. The vaccine for use according to any one of paragraphs 1-4, wherein each dose comprises 250 µg of ISC.
6. The vaccine for use according to any one of paragraphs 1-5, wherein the saponin is Quil A and the phospholipid is DPPC.
7. The vaccine for use according to paragraph 6, wherein the ratio of Quil A: DPPC: Cholesterol in the composition is 5:1:1 by weight.
8. The vaccine for use according to any one of paragraphs 1-7, wherein the second dose is administered 21-28 days after the first dose.

The disclosure encompasses an immunogenic composition comprising Hendra and/or Nipah virus G protein, an immunostimulatory complex (ISC) and one or more excipients in an amount effective to elicit production of neutralizing antibodies against the Hendra and/or Nipah virus following administration to a subject. In some embodiments of the disclosure, the immunogenic composition comprises a saponin, a phospholipid, and a steroid.

In some embodiments of the disclosure soluble Hendra virus G glycoprotein consists of amino acids 73 to 604 of the native Hendra G glycoprotein (SEQ ID NO:2). In some embodiments of the disclosure, the soluble Hendra virus G glycoprotein is encoded by a nucleotide sequence comprising nucleotides 64 to 1662 of SEQ ID NO: 16. In some embodiments of the disclosure, the soluble Hendra virus G protein is present in dimer form wherein each soluble Hendra virus G glycoprotein dimer subunit is connected by one or more disulfide bonds. In some embodiments of the disclosure, the soluble Hendra virus G protein is present in tetramer form. In some embodiments of the disclosure, the tetramer form exists as a dimer of dimers non-covalently linked and/or connected by one or more disulfide bonds. The concentration of soluble.Hendra virus G protein can be about 5 to 100 pg/ml in the immunogenic composition.

The saponin is isolated from *Quillaja saponaria* Molina and may be selected from QH-A, QH-B, QH-C or QS21. The phospholipid is selected from the group consisting of phosphatidyl choline (PC), dipalmitoyl phosphatidyl choline (DPPC), phosphatidic acid (phosphatidate) (PA), phosphatidylethanolamine (PE), phosphatidylserine (PS), phosphatidylinositol (PI) phosphatidylinositol phosphate (PIP), phosphatidylinositol bisphosphate (PIP2), phosphatidylinositol triphosphate (PIP3), phosphorylcholine (SPH), ceramide phosphorylethanolamine (Cer-PE) and ceramide phosphorylglycerol. In instances, the saponin is Quil A, the phospholipid is DPPC and the steroid is cholesterol and the ratio of Quil A: DPPC: cholesterol in the composition is 5:1:1 by weight.

The disclosure also encompasses a method of producing a neutralizing antibody response against a Hendra and/or Nipah virus in a subject comprising administering to the subject the immunogenic composition described herein in an amount and duration effective to produce the neutralizing antibody response. In some embodiments of the disclosure, the neutralizing antibody response reduces Hendra and/or Nipah virus reproduction in the subject and may also reduce Hendra and/or Nipah virus shedding in the subject. In some embodiments of the disclosure, the subject has been exposed to Hendra and/or Nipah virus while in other embodiments of the disclosure, the subject is suffering from a Hendra and/or Nipah virus infection. Also provided is a method of producing a neutralizing antibody response against a Hendra virus in a subject comprising administering to the subject the immunogenic composition described herein in an amount and duration effective to produce the neutralizing antibody response. Also provided is a method of producing a neutralizing antibody response against a Nipah virus in a subject comprising administering to the subject the immunogenic composition described herein in an amount and duration effective to produce the neutralizing antibody response. However, any reference to a method of treatment practised on the human or animal body is to be interpreted as compositions for use in such treatments.

In some instances of the disclosure, the immunogenic composition is administered intramuscularly. In some instances of the disclosure, the immunogenic composition is administered in multiple doses and the first dose is followed by a second dose at least about twenty-one days to about twenty-eight days after the first dose. In some embodiments of the disclosure, each dose contains about 50 or about 100 µg of soluble Hendra virus G protein.

The disclosure further encompasses a method of differentiating a subject vaccinated with the immunogenic composition described herein from a subject exposed to Hendra and/or Nipah virus comprising detecting the presence of an antibody in a biological sample isolated from the subject against at least one of any of the following HeV and/or NiV viral proteins selected from the group consisting of fusion protein (F), matrix protein (M), phosphoprotein (P), large protein (L) and nucleocapsid protein (N).

The immunogenic compositions and methods of the disclosure can be administered to a subject such as a human, horse, cow, sheep, pig, goat, chicken, dog or cat.

The disclosure also encompasses a method of producing a neutralizing antibody response against a Hendra and/or Nipah virus in a human subject comprising administering to the subject an immunogenic composition comprising a Hendra virus soluble G glycoprotein in an amount and duration effective to produce the neutralizing antibody response. In some instances of the disclosure, the immunogenic composition further comprises an adjuvant.

### Description of the Figures

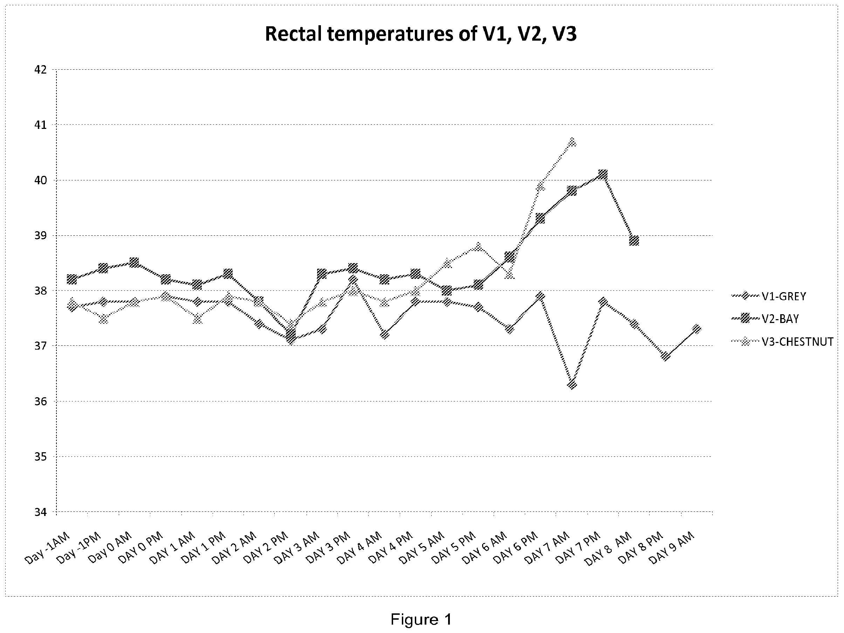

Figure 1 shows the rectal temperature over time for horses administered recombinant Hendra virus soluble glycoprotein (sG) at 50 or 100 µg/dose adjuvanted with 250 µg of immune stimulating complex followed by exposure to live Hendra virus at day 0.

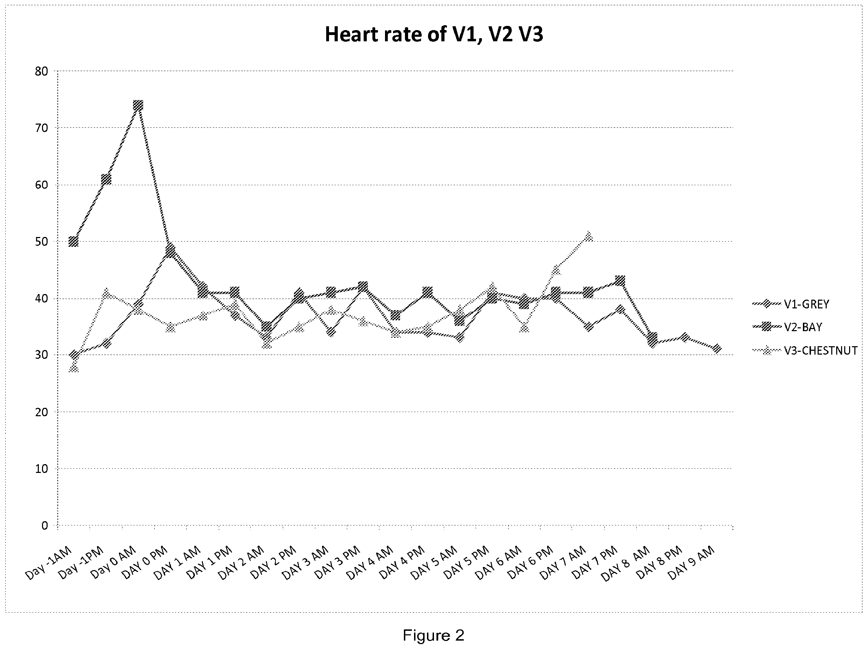

Figure 2 shows the heart rate over time for horses administered recombinant Hendra virus soluble glycoprotein (sG) at 50 or 100 µg/dose adjuvanted with 250 µg of immune stimulating complex followed by exposure to live Hendra virus at day 0.

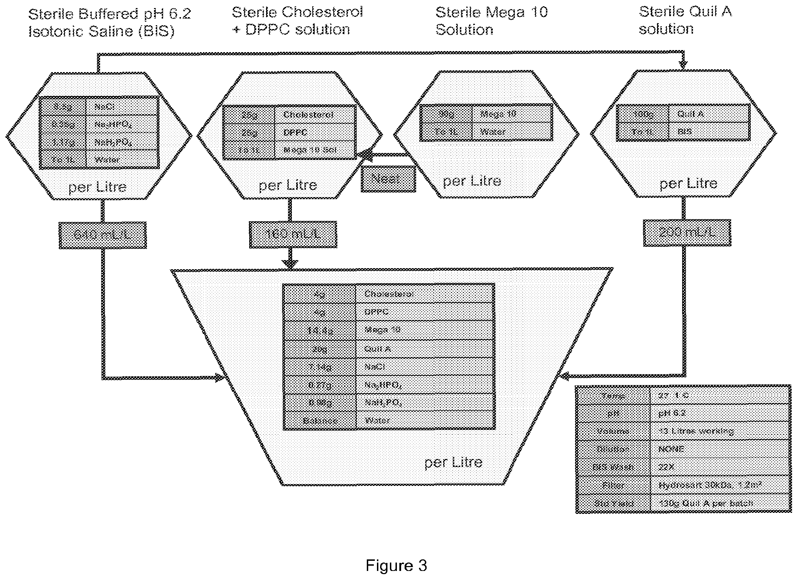

Figure 3 depicts a schematic for the preparation of an Immunostimulatory Complex.

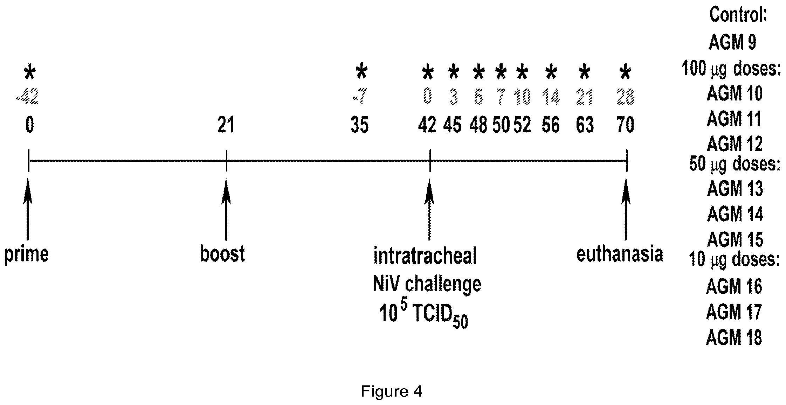

Figure 4 depicts a schematic diagram of sGHeV vaccination and NiV challenge schedule. Dates of sGHeV vaccination, NiV challenge and euthanasia are indicated by arrows. Blood and swab specimens were collected on days -42, -7, 0, 3, 5, 7, 10, 14, 21 and 28 post-challenge as indicated (*). Gray text denotes challenge timeline (top row); black text denotes vaccination timeline (bottom row). African green monkey (AGM) number for subjects in each vaccine dose group and one control subject are shown.

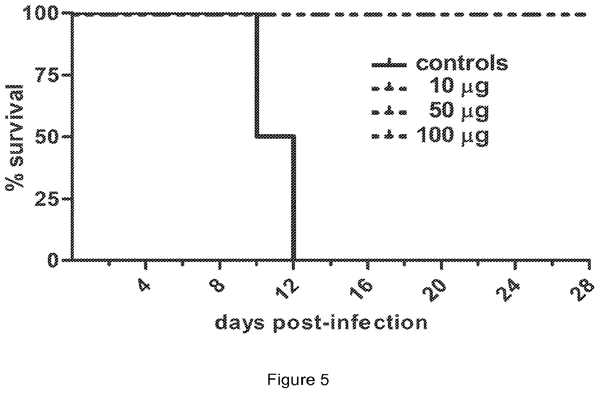

Figure 5 depicts the survival curve of NiV-infected subjects. Data from control subjects (n=2) and sGHeV vaccinated subjects (n=9) were used to generate the Kaplan-Meier survival curve. Control included data from one additional historical control subject. Vaccinated subjects received 10 µg, 50 µg or 100 µg sGHeV administered subcutaneously twice. Average time to end stage disease was 11 days in control subjects whereas all vaccinated subjects survived until euthanasia at the end of the study.

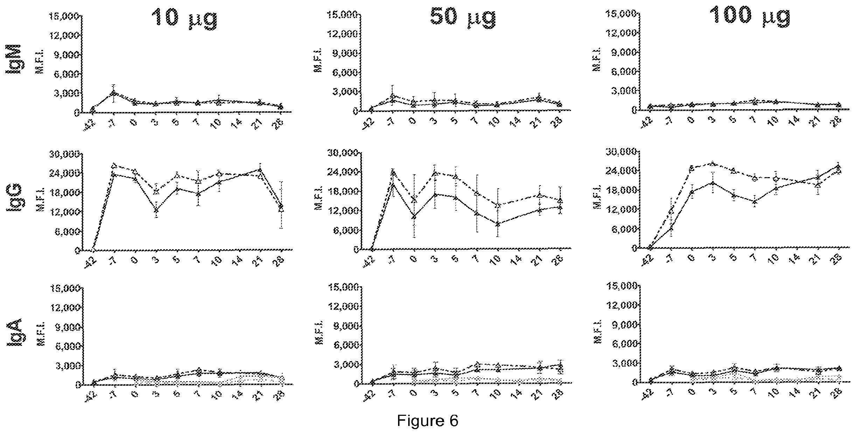

Figure 6 depicts NiV- and HeV- specific Immunoglobulin (Ig) in vaccinated subjects. Serum and nasal swabs were collected from vaccinated subjects and IgG, JgA and IgM responses were evaluated using sGHeV, and sGNiV multiplexed microsphere assays. Sera or swabs from subjects in the same vaccine dose group (n=3) were assayed individually and the mean of microsphere median fluorescence intensities (M.F.I.) was calculated which is shown on the Y-axis. Error bars represent the standard error of the mean. Serum sG-specific Ig is shown in black (sGHeV (open triangles), sGNiV (solid triangles)) and mucosal sG-specific IgA is shown in gray symbols (sGHeV (open triangles), sGNiV (solid triangles)).

### Description of the Disclosure

### Vaccine & Immunogenic Compositions

The vaccine and immunogenic composition of the present disclosure induces at least one of a number of humoral and cellular immune responses in a subject who has been administered the composition or is effective in enhancing at least one immune response against at least one strain of HeV and/or NiV, such that the administration is suitable for vaccination purposes and/or prevention of HeV and/or NiV infection by one or more strains of HeV and/or NiV. The composition of the present disclosure delivers to a subject in need thereof a G glycoprotein, including soluble G glycoproteins from HeV and/or NiV and an Immunostimulatory Complex (ISC) which acts as an adjuvant. In some embodiments of the disclosure, the amount of G glycoprotein includes, but is not limited to, 5, 10, 15, 20, 25, 30, 35, 40, 45, 50, 75, 100, 150, 200 or 250 µg per ml which can also contain 100, 125, 150, 175, 200, 225, 250, 275 or 300 µg per ml of ISC. In some embodiments of the disclosure, the amount of G glycoprotein is 5, 50 or 100 and the amount of ISC is 250 µg per ml.

### A. HeV and NiV G proteins

In some embodiments of the disclosure, the vaccine and immunogenic compositions comprise one or more HeV and/or NiV G glycoproteins as described herein. The term protein is used broadly herein to include polypeptide or fragments thereof. By way of example, and not limitation, a HeV G glycoprotein may in soluble form and comprise amino acids 73-604 of the amino acid sequence for a HeV G glycoprotein in Wang (2000) J. Virol. 74, 9972-9979 (see also Yu (1998) Virology 251, 227-233). Also by way of example and not limitation, a NiV G glycoprotein may be in soluble form and comprise amino acids 71-602 of the amino acid sequence for a NiV G glycoprotein in Harcourt (2000) Virology 271: 334-349, 2000 (see also Chua (2000) Science, 288, 1432-1).

Generally, the soluble forms of the HeV and NiV G glycoproteins comprise all or part of the ectodomain (e.g. extracellular) of the G glycoprotein of a HeV or NiV and are generally produced by deleting all or part of the transmembrane domain of the G glycoprotein and all or part of the cytoplasmic tail of the G glycoprotein. By way of example, a soluble G glycoprotein may comprise the complete ectodomain of a HeV or NiV G glycoprotein. Also by way of example, and not limitation a soluble G glycoprotein may comprise all or part of the ectodomain and part of the transmembrane domain of a HeV or NiV G glycoprotein.

The soluble HeV or NiV G glycoproteins of the disclosure, generally retain one or more characteristics of the corresponding native viral glycoprotein, such as, ability to interact or bind the viral host cell receptor, can be produced in oligomeric form or forms, or the ability to elicit antibodies (including, but not limited to, viral neutralizing antibodies) capable of recognizing native G glycoprotein. Examples of additional characteristics include, but are not limited to, the ability to block or prevent infection of a host cell. Conventional methodology may be utilized to evaluate soluble HeV or NiV G glycoproteins for one of more of the characteristics.

By way of example, and not limitation, a polynucleotide encoding a soluble HeV G glycoprotein may comprise a polynucleotide sequence encoding about amino acids 73-604 of the amino acid sequence for an HeV G glycoprotein in Wang (2000) J. Virol. 74, 9972-9979 (SEQ ID NO: 2). Also by way of example, and not limitation, a polynucleotide encoding a soluble HeV G glycoprotein may comprise nucleotides 9129 to 10727 of the polynucleotide sequence for an HeV G glycoprotein in Wang (2000) J. Virol. 74, 9972-9979. In addition, codon optimized polynucleotide sequence encoding about amino acids 73-604 of the amino acid sequence fpr an HeV G glycoprotein (SEQ ID NO: 2) can also be utilized. In some embodiments of the disclosure, these codon optimized sequences comprises or consist of nucleotides 64 to 1662 of SEQ ID NO: 16. In further embodiments of the disclosure, the codon optimized sequences comprises or consists of SEQ ID NO: 16 which includes nucleotides encoding an Igk leader sequence.

By way of example, and not limitation, a NiV G glycoprotein may in soluble form and comprise amino acids 71-602 of the amino acid sequence for the NiV G glycoprotein in Harcourt (2000) Virology 271, 334-349. Non-limiting examples of sequences that may be used to construct a soluble NiV G glycoprotein can be found in Harcourt (2000) Virology 271, 334-349, Generally, G glycoprotein sequences from any Nipah virus isolate or strain may be utilized to derive the polynucleotides and polypeptides of the disclosure.

By way of example, and not limitation, a polynucleotide encoding a soluble NiV G glycoprotein may comprise a polynucleotide sequence encoding about amino acids 71-602 of the amino acid sequence for an NiV G Glycoprotein in Harcourt (2000) Virology 271, 334-349. Also by way of example, and not limitation, a polynucleotide encoding a soluble NiV G glycoprotein may comprise 234-2042 of the polynucleotide sequence for an NIV G glycoprotein in Harcourt (2000) Virology 271, 334-349 (SEQ ID NO: 4). in addition, codon optimized polynucleotide sequence encoding about amino acids 71-602 of the amino acid sequence for an NiV G glycoprotein can also be utilized.

Functional equivalents of these G glycoproteins can be used in the immunogenic and vaccine compositions of the disclosure. By way of example and not limitation functionally equivalent polypeptides possess one or more of the following characteristics: ability to interact or bind the viral host cell receptor, can be produced in dimeric or tetrameric form or forms, the ability to elicit antibodies (including, but not limited to, HeV and/or NiV viral neutralizing antibodies) capable of recognizing native G glycoprotein and/or the ability to block or prevent infection of a host cell.

In some embodiments of the disclosure, the G glycoprotein may be in dimeric and/or tetrameric form. Such dimers depend upon the formation of disulfide bonds formed between cysteine residues in the G glycoprotein. Such disulfide bonds can correspond to those formed in the native G glycoprotein (e.g. location of cyteines remains unchanged) when expressed in the surface of HeV or NiV or may be altered in the presence or location (e.g. by altering the location of cysteine(s) in the amino acid sequence) of the G glycoprotein so as to form different dimeric and/or tetrameric forms of the G glycoprotein which enhance antigenicity. Additionally, non-dimerized and tetramerized forms are also within the disclosure, again taking into account that G glycoprotein presents numerous conformation-dependent epitopes (i.e. that arise from a tertiary three dimensional structure) and that preservation numerous of such natural epitopes is highly preferred so as to impart a neutralizing antibody response.

The HeV immunogenic and vaccine compositions of the disclosure may contain proteins of variable length but include the amino acid residues 73 to 604 of SEQ ID NO: 2. In one embodiment of the present disclosure, envelope proteins of the disclosure are at least about 85, 90, 91, 92, 93, 94, 95 , 96, 97, 98, or 99% identical to the HeV glycoprotein of SEQ ID NO: 2 (including amino acids 73 to 604). Accordingly, the HeV G glycoproteins of the disclosure comprise immunogenic fragments of the native HeV G glycoprotein with sufficient number of amino acids to produce conformational epitopes. Non-limiting examples of immunogenic fragments include amino acid sequences which may be at least 530, 531, 532, 533, 534 or 535 or more amino acids in length. In some embodiments of the disclosure, the HeV G glycoprotein comprises or consists of SEQ ID NO: 2 or synthetic constructs further comprising an Igk leader sequence (SEQ ID NO: 15).

The NiV immunogenic and vaccine compositions of the disclosure may contain proteins of variable length but include the amino acid residues 71 to 602 of SEQ ID NO: 4. In one embodiment of the present disclosure, envelope proteins of the disclosure are at least about 85, 90, 91, 92, 93, 94, 95, 96, 97, 98, or 99% identical to the NiV glycoprotein of SEQ ID NO: 4 (including amino acids 71 to 602). Accordingly, the NiV G glycoproteins of the disclosure comprise immunogenic fragments of the native NiV G glycoprotein with sufficient number of amino acids to produce conformational epitopes. Non-limiting examples of immunogenic fragments include amino acid sequences which may be at least 528, 529, 530, 531, 532, or 533 or more amino acids in length. In some embodiments of the disclosure, the NiV G glycoprotein comprises or consists of SEQ ID NO: 4 or synthetic constructs further comprising a leader sequence.

Immunogenic fragments as described herein will contain at least one epitope of the antigen and display HeV and/or NiV antigenicity and are capable of raising an immune response when presented in a suitable construct, such as for example when fused to other HeV and/or NiV antigens or presented on a carrier, the immune response being directed against the native antigen. In one embodiment of the present disclosure, the immunogenic i fragments contain at least 20 contiguous amino acids from the HeV and/or NiV antigen, for example, at least 50, 75, or 100 contiguous amino acids from the HeV and/or NiV antigen.

HeV and NiV G glycoprotein embodiments of the disclosure further include an isolated polypeptide comprising an amino acid sequence having at least a 85, 90, 91, 92, 93, 94, 95, 96, 97, 98, 99 or 100% identity to native HeV or NiV G glycoproteins, wherein said polypeptide sequence may be identical to the native HeV or NiV G glycoprotein amino acid sequence or may include up to a certain integer number of amino acid alterations as compared to the native HeV or NiV G protein amino acid sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the native HeV or NiV G glycoprotein amino acid sequence.

Sequence identity or homology at the amino acid sequence level can be determined by BLAST (Basic Local Alignment Search Tool) analysis using the algorithm employed by the programs blastp, blastn, blastx, tblastn and tblastx (Altschul (1997) Nucleic Acids Res. 25, 3389-3402 and Karlin (1990) Proc. Natl. Acad. Sci. USA 87, 2264-2268) which are tailored for sequence similarity searching. The approach used by the BLAST program is to first consider similar segments, with gaps (non-contiguous) and without gaps (contiguous), between a query sequence and a database sequence, then to evaluate the statistical significance of all matches that are identified and finally to summarize only those matches which satisfy a preselected threshold of significance. For a discussion of basic issues in similarity searching of sequence databases, see Altschul (1994) Nature Genetics 6, 119-129. The search parameters for histogram, descriptions, alignments, expect (i.e., the statistical significance threshold for reporting matches against database sequences), cutoff, matrix and filter (low complexity) are at the default settings. The default scoring matrix used by blastp, blastx, tblastn, and tblastx is the BLOSUM62 matrix (Henikoff (1992) Proc. Natl. Acad. Sci. USA 89, 10915-10919), recommended for query sequences over 85 amino acids in length.

The vaccine and immunogenic compositions of the present disclosure may further comprise additional HeV and/or NiV G proteins from different strains that may further potentiate the immunization methods of the disclosure.

### B. Immunostimulatory Complexes

Generally this disclosure provides immunogenic compositions, including vaccine compositions, comprising soluble forms of HeV and/or NiV G glycoprotein envelope protein in combination with an immune stimulatory complex (ISC) and to methods for using these compositions for preventing and treating HeV and/or NiV infections in a subject. In the present disclosure, the vaccine and/or immunogenic composition comprise an immunostimulatory complex which acts as an adjuvant. As used herein, "adjuvant" refers to an agent which, while not having any specific antigenic effect in itself, may stimulate the immune system, increasing the response to an antigen.

ISC have a number of features that makes it an ideal adjuvant for certain applications:
Antigen sparing: As noted for example in Wee (2008) Mucosal Immunol. 1, 489-496 in situations where antigen availability is limited or antigen costs are high, ISC has been shown to allow antigen sparing as much as 10 to 100 fold. Most likely this due to a combination of increased efficiency or more appropriate mechanism of action compared to other adjuvants.

Cross-presentation: As noted for example in Schnurr (2009) J. Immunol. 182, 1253-1259, presentation of antigen by antigen presenting cells (APCs) usually follows one of two pathways. Foreign antigen is usually engulfed by APCs and then processed and reexpressed on the surface of APC in the context of Major Histocompatibility Complex (MHC) class II molecules. They are then able to be seen by lymphocytes and, if the right co-stimulatory factors/signals are present, be responded to as appropriate. Self or cancer antigens and viral antigens are normally processed and expressed in the context of Class I molecules as they are present in the cytoplasm of APCs. Effective immunity to cancer and viral antigens requires access to the Class I pathway. This occurs naturally during viral infection or cellular homeostasis (cellular turnover of internal antigens). Antigens (viral or self) introduced as vaccines need to find their way from outside the cell to antigen processing machinery of the cell and entry into the Class II pathway to the Class I pathway. This can occur naturally in Dendritic Cells (DCs - specialist APCs) or can be achieved by vaccinating with antigens mixed with ISC as adjuvant. This process of externally derived antigen finding its way into the Class I pathway of antigen presentation is called cross-presentation. The precise mechanism by which ISC achieves cross-presentation of antigen has not been fully elucidated but may rely on membrane perturbation of ISC components.

Humoral and cell mediated responses: As noted, for example in Maraskovsky (2009) Immunol. Cell Biol. 87, 371-376), by virtue of the mechanism of action of ISC both humoral and cellular arms of the adaptive immune system are engaged. In some species this is paralleled the profile of cytokines stimulated by vaccination with this adjuvant. Type 1 immune responses characterized by Interleukin-2 and IFN-gamma expression and protection against intracellular pathogens (bacteria, protozoa and viruses) and type 2 responses characterized by expression of Interleukin-4 and generation of neutralizing antibody for antitoxin and anti-pathogen related immunity. ISC provides a balanced cytokine profile between these two extremes allowing for greater breadth of immune response. Additionally, a number of studies have shown that ISC can be effective if vaccines are delivered intranasally. This allows for sensitization of mucosal surfaces and thus providing relevant immunity at the site of pathogen entry, of particular relevance in this case (mucosal immunity), see also Sjölander (2001) Vaccine 19, 4072-4080.

Sterile filterable and consistent manufacturing criteria: The size of the ISC particle is routinely 40 nm in diameter allowing it to pass through filters used to sterilize preparations late in formulation. Additionally, the natural tendency for triterpenoid saponins as found in Quil A to associate with cholesterol and phospholipids has been taken advantage of in developing manufacturing methods for ISC. Quil A species that do not form ISC particles are dialyzed away from the final product. By controlling ratios of the components a consistent product is generated from a heterogeneous spectrum of Quil A saponins. This ratio is important as deviation leads to structures that are not characteristic 40 nm particles (helices, sheets etc.). The free-flowing nature of the ISC colloid and its ability to be measured using transmission electron microscopy, HPLC and other techniques make this adjuvant amenable to development of release assays and other measures of quality.

Thus, based on the above, in some embodiments of the disclosure, the formulation of an immunostimulating complex with an optimal amount of G glycoprotein includes a saponin, a phospholipid and a steroid molecule. In some embodiments of the disclosure, the molar ratio of saponin, phospholipid, steroid molecule in a ratio of 5:1:1. An immunostimulating complex may contain, for example, 5 to 10% by weight saponin, 1 to 5% steroid molecule and phospholipid and the remainder comprising G glycoprotein. G glycoprotein can be incorporated into the immunostimulating complex either directly or by chemical coupling to a carrier protein (e.g. chimeric or fusion protein) after incorporation of protein into immunostimulating complexes. Reference to an immunostimulating complex should be understood to include reference to derivatives, chemical equivalents and analogs thereof In some embodiments of the disclosure, the ISC is admixed separately from the HeV and/or NiV G glycoprotein then the G glycoprotein is admixed with the ISC. In some embodiments of the disclosure, the G glycoprotein is admixed directly with the saponin, phospholipid and steroid molecule.

In some embodiments described herein, the saponin for use in the present invention may be Quil A and/or its derivatives. Quil A is a saponin preparation isolated from the South American tree *Quillaja saponaria* Molina and was first described as having adjuvant activity by Dalsgaard (1974) Saponin adjuvants, Archiv. für die gesamte Virusforschung, Vol. 44, Springer Verlag, pp. 243-254. Purified fragments of Quil A have been isolated by HPLC which retain adjuvant activity without the toxicity associated with Quil A (EP 0362278), for example QS7 and QS21 (also known as QA7 and QA21). QS21 is a natural saponin derived from the bark of *Quillaja saponaria* Molina which induces CD8t cytotoxic T cells (CTL), Th1 cells and a predominant IgG2a antibody response and is a saponin for use in the context of the present invention. Other suitable saponins for use in the ISC include, but are not limited to, the QH-A, QH-B and QH-C subtractions of Quil A, those from species other than *Quillaia saponaria* such as those from the genera *Panax* (ginseng), *Astragalus, Achyranthes,* Soy bean, Acacia and *Codonopsis.* In some embodiments, the saponin is isolated from a species other than *Quillaia saponaria.*

Non-limiting examples of phospholipids for use in the immunogenic and vaccine compositions of the disclosure include molecules with diacylglyceride structures and phosphosphingolipids. Non-limiting examples of phospholipids with diacyglyceride structures include phosphatidic acid (phosphatidate) (PA), phosphatidylethanolamine (cephalin) (PE), phosphatidylcholine (lecithin) (PC), dipalmitoyl phosphatidylcholine (DPPC) or phosphatidylserine (PS). Another non-limiting example of phospholipids with diacylgylceride structures includes phosphoinositides. Exemplary phosphoinositides include, but are not limited to, phosphatidylinositol (PI), phosphatidylinositol phosphate (PIP), phosphatidylinositol bisphosphate (PIP2) or phosphatidylinositol triphosphate (PIP3). Non-limiting examples of phosphospingolipids include, ceramide phosphorylcholine (Sphingomyelin) (SPH), ceramide phosphorylethanolamine (Sphingomyelin) (Cer-PE) or ceramide phosphorylglycerol.

Steroid molecules for use in the immunogenic and vaccine compositions of the disclosure include molecules which incorporate a steroid as part of their structure. Non-limiting examples of steroid molecules include cholesterol, pregnenolone, 17-alpha-hyrdroxy pregnenolone, dehydroepiandrosterone, androstenediol, progesterone, 17-alpha-hydroxy progesterone, androstenedione, testosterone, dihyrdroxytestorone, deoxycorticosterone, 11-deoxycorticosterone, cortisol, corticosterone, aldosterone, estrone, estradiol or estriol.

In some embodiments of the disclosure, immunostimulating complexes are typically, but not limited to, small cage like structures 30-40 nM in diameter. In some embodiments of the disclosure, the formulation of an immunostimulating complex has a molar ratio of Quil A, cholesterol, phosphatidylcholine and G glycoprotein in a ratio of 5:1:1. An immunostimulating complex may contain, for example, 5 to 10% by weight Quil A, 1 to 5% cholesterol and phospholipids and the remainder comprising G glycoprotein. G glycoprotein can be incorporated into the immunostimulating complex either directly or by coupling to a carrier protein (e.g. a chimeric or fusion protein) after incorporation of protein into immunostimulating complexes. Reference to an immunostimulating complex should be understood to include reference to derivatives, chemical equivalents and analogs thereof. For example, reference to a derivative of an immunostimulating complex includes reference to an immunostimulating complex in which one or more of Quil A, cholesterol, phosphatidylcholine or protein, for example, are deleted, substituted for, or where a component in addition to Quil A, cholesterol, phosphatidylcholine or protein is added to the complex. The functional equivalent of an immunostimulating complex may be an immunostimulating complex in which one or more of its four components are replaced with a functional equivalent. In some embodiment of the present disclosure, the G glycoprotein component of the Immunostimulating complex is deleted. This type of immunostimulating complex is herein referred to as a protein-free immunostimulating complex.

In some embodiments the present disclosure includes, but is not limited to, an immunogenic composition comprising an isolated HeV or NiV G protein capable of inducing the production of a cross-reactive neutralizing anti-serum against multiple strains of HeV and/or NiV in vitro and an adjuvant comprising Quil A, DPPC and cholesterol, for example wherein the composition contains: 5, 50 or 100 µg of soluble HeV or NiV G protein, and appropriate amounts of Quil A, DPPC, and cholesterol. Further examples of immunostimulatory complexes, and the preparation thereof, are described in EP 024238081 and EP 0180564B1, and also WO2000041720 (see, for example, pages 3 and 9 therein, referring to: Cox & Coulter (1992) Advances in Adjuvant Technology and Application in Animal Parasite Control Utilizing Biotechnology, Chapter 4, Yong (ed.), CRC Press; Dalsgard (1974) Gesamte Virusforsch, 44, 243-254; Australian Patent Specification Nos. 558258, 589915, 590904 & 632067. See also the representative protocols described in U.S. Patent 6,506,386, and reference therein to the well known fact that immunostimulatory complexes can be used wherein the protein antigen is included in the immunostimulatory complex when formed (see EP 0109942B1), or alternatively, preformed immunostimulatory complexes are provided which are then mixed with a separately added aliquot of antigen to form the vaccine (see EP 0436620B1). As will be generally recognized, the protein antigen can also be covaltently attached to the immunostimulatory complex (see again EP 0180564B1). As is also well recognized in the art, immunostimulatory complexes may be administered via muscosal vaccination (see Mowat (1991) Immunology 72, 317-322) and immunostimulatory complexes of the present disclosure may be further improved for muscosal vaccination by inclusion of membrane targeting proteins (WO 9730728).

In some embodiments the present disclosure includes, but is not limited to, an immunogenic composition comprising an isolated HeV or NiV G protein capable of inducing the production of a cross-reactive neutralizing anti-serum against multiple strains of HeV and/or NiV in vitro and an adjuvant comprising Quil A, DPPC and cholesterol, for example wherein the composition contains: 5, 50 or 100 µg of soluble HeV or NiV G protein, and appropriate amounts of Quil A, DPPC, and cholesterol. Further examples of immunostimulatory complexes are described in WO2000041720.

in another embodiment of the disclosure, the vaccine and immunogenic compositions may be part of a pharmaceutical composition. The pharmaceutical compositions of the present disclosure may contain suitable pharmaceutically acceptable carriers comprising excipients and auxiliaries that facilitate processing of the active compounds into preparations that can be used pharmaceutically for delivery to the site of action.

### C. Excipients

The vaccine compositions of the invention can further comprise pharmaceutically acceptable carriers, excipients and/or stabilizers (see e.g. Remington: The Science and practice of Pharmacy (2005) Lippincott Williams), in the form of lyophilized formulations or aqueous solutions. Acceptable carriers, excipients, or stabilizers are nontoxic to recipients at the dosages and concentrations, and may comprise buffers such as phosphate, citrate, and other organic acids; antioxidants including ascorbic acid and methionine; preservatives (such as Mercury((o-carboxyphenyl)thio)ethyl sodium salt (THIOMERSAL), octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG), TWEEN or PLURONICS.

The compositions of the invention can be in dosages suspended in any appropriate pharmaceutical vehicle or carrier in sufficient volume to carry the dosage. Generally, the final volume, including carriers, adjuvants, and the like, typically will be at least 1.0 ml. The upper limit is governed by the practicality of the amount to be administered, generally no more than about 0.5 ml to about 2.0 ml.

### Methods of Use

The disclosure encompasses methods of preventing and/or treating Hendra and/or Nipah virus infection comprising administering the immunogenic and vaccine compositions of the disclosure in any mammalian subject. Active immunity elicited by vaccination with a HeV and/or NiV G glycoprotein with the adjuvants described herein can prime or boost a cellular or humoral immune response. An effective amount of the HeV and/or NiV G glycoprotein or antigenic fragments thereof can be prepared in an admixture with an adjuvant to prepare a vaccine.

' The disclosure encompasses methods of preventing and/or treating Hendra and/or Nipah virus infection in a human subject comprising administering an immunogenic and/or vaccine composition comprising a soluble HeV and/or NiV G glycoprotein or combinations thereof either by itself or in combination with at least one adjuvant suitable for use in humans. Adjuvants suitable for use in humans may be used alone or in combination. Examples of adjuvants suitable for use in humans include, but are not limited to, aluminum salts. Examples of aluminum salts include, but are not limited to, aluminum hydroxide, aluminium hydroxide gel (Alhydrogel^{™}), aluminum phosphate, alum (potassium aluminum sulfate), or mixed aluminum salts. Additional examples of adjuvants suitable for use in humans include, but are not limited to, water-in-oil emulsions, oil-in-water emulsions, and AS04 (combination of aluminum hydroxide and monophosphoryl lipid A) and CpG oligodeoxynucleotides. CpG oligodeoxynucleotides are synthetic oligonucleotides that contain unmethylated CpG dinucleotides in particular sequence contexts (CpG motifs). These CpG motifs are present at a 20-fold greater frequency in bacterial DNA compared to mammalian DNA. CpG oligodeoxynucleotides are recognized by Toll-like receptor 9 (TLR9) leading to strong immunostimulatory effects.

The administration of a vaccine or immunogenic composition comprising HeV and/or NiV G glycoprotein with one or more adjuvants described herein, can be for either a prophylactic or therapeutic purpose. In one aspect of the present disclosure the composition is useful for prophylactic purposes. When provided prophylaqtically, the vaccine composition is provided in advance of any detection or symptom of HeV and/or NiV infection. The prophylactic administration of an effective amount of the compound(s) serves to prevent or attenuate any subsequent HeV and/or NiV infection.

When provided therapeutically, the vaccine is provided in an effective amount upon the detection of a symptom of actual infection. A composition is said to be "pharmacologically acceptable" if its administration can be tolerated by a recipient. Such a composition is said to be administered in a "therapeutically or prophylactically effective amount" if the amount administered is physiologically significant. A vaccine or immunogenic composition of the present disclosure is physiologically significant if its presence results in a detectable change in the physiology of a recipient patient, for example, by enhancing a broadly reactive humoral or cellular immune response to one or more strains of HeV and/or NiV. The protection provided need not be absolute (i.e., the HeV or NiV infection need not be totally prevented or eradicated), provided that there is a statistically significant improvement relative to a control population. Protection can be limited to mitigating the severity or rapidity of onset of symptoms of the disease.

A vaccine or immunogenic composition of the present disclosure can confer resistance to multiple strains of HeV and/or NiV. As used herein, a vaccine is said to prevent or attenuate an infection if its administration to a subject results either in the total or partial attenuation (i.e., suppression) of a symptom or condition of the infection, or in the total or partial immunity of the individual to the infection.

At least one vaccine or immunogenic composition of the present disclosure can be administered by any means that achieve the intended purpose, using a pharmaceutical composition as described herein. For example, administration of such a composition can be by various parenteral routes such as subcutaneous, intravenous, intradermal, intramuscular, intraperitoneal, intranasal, transdermal, or buccal routes. In one embodiment of the present disclosure, the composition is administered by subcutaneously. Parenteral administration can be by bolus injection or by gradual perfusion over time.

A typical regimen for preventing, suppressing, or treating a disease or condition which can be alleviated by a cellular immune response by active specific cellular immunotherapy, comprises administration of an effective amount of a vaccine composition as described above, administered as a single treatment, or repeated as enhancing or booster dosages, over a period up to and including one week to about twenty-four months. Non-limiting examples include a first dose followed by a second dose about at least 10, 11, 12, 13, 14, 15, 16, 17, 18, 19, 20, 21, 22, 23 or 24 days after the first dose (day 0). The amount of the dose of the immunogenic or vaccine composition may be the less than, the same as, or greater than the first dose administered at day 0.

According to the present disclosure, an "effective amount" of a vaccine or immunogenic composition is one which is sufficient to achieve a desired biological effect, in this case at least one of cellular or humoral immune response to one or more strains of HeV and/or NiV. It is understood that the effective dosage will be dependent upon the age, sex, health, and weight of the subject, kind of concurrent treatment, if any, frequency of treatment, and the nature of the effect desired. The ranges of effective doses provided below are not intended to limit the disclosure and represent examples of dose ranges which may be suitable for administering compositions of the present disclosure. However, the dosage may be tailored to the individual subject, as is understood and determinable by one of skill in the art, without undue experimentation.

The recipients of the vaccine and immunogenic compositions of the present disclosure, can be any subject which can acquire specific immunity via a cellular or humoral immune response to HeV and/or NiV, where the cellular response is mediated by an MHC class i or class ii protein. Among mammals, the recipients may be mammals of the orders primata (including humans, chimpanzees, apes and monkeys). In one embodiment of the present disclosure there is provided a method of treating humans with the vaccine or immunogenic compositions of the disclosure. The subjects may be infected with HeV and/or NiV or provide a model of HeV or NiV infection as in experimental studies. In some embodiments of the disclosure, the subject is a domesticated mammal including, but not limited to, a horse, cow, oxen, water buffalo, sheep, pig (Mingyi (2010) Vet. Res. 41, 33), goat, dog (Biosecurity Alert - Hendra Virus Update, 27 July 2011, Press Release, Biosecurity Queensland) or cat. In some embodiments of the disclosure, the subject is a fowl, including a chicken.

Vaccines of the present invention also provide for cross-protection against Nipah virus infection at doses used to protect against Hendra virus infection and thus also provide effective vaccination against Nipah virus.

Reference to an effective immune response should be understood as a reference to an immune response which either directly or indirectly leads to a beneficial prophylactic or therapeutic effect. In the case where the immunogen comprises a HeV or NiV G glycoprotein as described herein, such a response includes the reduction or blocking of viral reproduction and/or viral shedding and/or reduction in disease symptoms in an animal. It should be understood that efficacy is a functional measure and is not defined by reference to anti-HeV and/or anti-NiV antibody titre alone since the presence of circulating antibody alone is not necessarily indicative of the capacity of said circulating antibody to block viral reproduction and shedding.

Also by way of example, and not limitation, if a soluble G protein polypeptide of the disclosure is being administered to augment the immune response in a subject infected with or suspected of being infected with Hendra of Nipah and/or if antibodies of the present disclosure are being administered as a form of passive immunotherapy the composition can further comprise, for example, other therapeutic agents (e.g., anti-viral agents).

Example 4 below provides information on certain preferred compositions for use in vaccinating horses. In regard of other animals that may be infected with Hendra virus, and which therefore warrant vaccination to protect both animals and thus humans from both Hendra and Nipah virus infection, the following information is generally applicable and can readily be adapted by those skilled in the art. Generally speaking, companion animals (dogs and cats) warrant approximately 25 micrograms of Hendra antigen, and can benefit from an ISC adjuvant in the range of 25-150 micrograms, with a 5:1:1 ratio of saponin, phospholipid and sterol being among the preferred ISC compositions while using any of the component species as disclosed herein. For companion animals it is preferred that the final dose be about 1 ml. Polygen^{™} (MVP Technologies), a copolymer based adjuvant, may also be used at preferably about 5-15% (v/v).

Generally speaking, for larger farm animals (sheep, cows, pigs, etc.) the antigen and adjuvant dosing (and final dosing volume) amounts otherwise provided herein for horses are applicable, that is, from 50-100 micrograms of antigen, and typically about 250 micrograms of ISC may be used, final volume 1-3 ml for example). In regard of pigs, an alternative and effective adjuvant formulation involves (for approximately the same amount of antigen) a blend of ISC and ionic polysaccharide, specifically 100 mg DEAE dextran and 800 micrograms ISC in 1-3 ml final dose volume (again 5:1:1 of Quil A:phoshatidyl choline:cholesterol (see WO 2000/41720)).

### Differentiation of Vaccinated Animals

The disclosure also encompasses methods of differentiating healthy vaccinated animals from animals exposed to, or infected with HeV and/or NiV. During viral infection, HeV and NiV express additional proteins other than G glycoprotein (G) Including fusion protein (F), matrix protein (M), phosphoprotein (P), large protein (L) and nucleocapsid protein (N). These additional proteins have the potential to induce immune responses in animals in the form of antibodies which bind to these proteins or T cell immunity. The level of antibody response to these other proteins can normally be measured by assays such as enzyme-linked immune assay (EIA). The immunogenic and vaccine formulations of the present disclosure, in some embodiments, contain only G glycoprotein as an HeV and/or NiV antigen and will therefore induce immune responses with antibodies only to the G glycoprotein of HeV and/or NiV. Animals vaccinated with the immunogenic compositions described herein which are subsequently infected by HeV or NiV will mount a booster immune response to the G glycoprotein, but will also show changes of antibody presentation to some other HeV and NiV proteins other than G glycoprotein. Thus, the presence of antibodies to any of the fusion protein (F), matrix protein (M), phosphoprotein (P), large protein (L) and nucleocapsid protein (N) can be measured in an EIA to determine the presence or absence of antibodies specific to these proteins in serum samples. If antibody to any of these other proteins (i.e. other than G glycoprotein) is detected, then the animal has been exposed to HeV and/or NiV. Alternatively, if no antibody to these other proteins is found and only antibodies binding to G protein are detected, then the animal has only been vaccinated.

The EIA of the present disclosure are both highly specific and highly selective in detecting and differentiating between animals infected with HeV and/or NiV and healthy animals which have been vaccinated with the immunogenic compositions described herein. The present disclosure may utilize a variety of assay procedures including ELISA in both homogenous and heterogenous environments. The assay procedures may be conducted on samples such as blood, serum, mtlk, or any other body fluid containing antibodies.

In some embodiments of the disclosure, the antibodies used in the EIA may uniquely compete with antibodies induced by vaccination with the G glycoprotein, but not antibodies induced in animals by infection with HeV and/or NiV. This allows not only serologic diagnosis of HeV and NiV infection, but differentiation of vaccination from infection in a single assay. The EIA procedure may be performed on standard blood serum samples or any body fluids or secretions containing antibodies. The EIA procedure may employ either monoclonal and/or polyclonal antibodies to G glycoprotein and any other HeV and/or NiV viral protein (e.g. fusion protein (F), matrix protein (M), phosphoprotein (P), large protein (L) and nucleocapsid protein (N) as such proteins are not present in vaccinated healthy animals which have not been exposed to HeV and/or NiV). The EIA may be carried out in any number of commercially available fixed or portable-manual, semi-automated or robotics-automated ELISA equipment with or without computer assisted data analysis reduction software and hardware. In some embodiments of the disclosure the methods of differentiating healthy vaccinated animals from animals exposed to, or infected with HeV and/or NiV may be conducted on a biological sample isolated from a domesticated mammal including, but not limited to, a horse, cow, sheep, pig, goat, dog or cat. In some embodiments of the disclosure, the subject is a fowl, including a chicken. In some embodiments of the disclosure, the subject is a human.

### Examples

### Example 1: Vector Constructs

Vectors were constructed to express transmembrane/cytoplasmic tail-deleted HeV G or NiV G. The cloned cDNA of full-length HeV or NiV G protein were amplified by PCR to generate fragments about 2600 nucleotides encoding the transmembrane domain/cytoplasmic tail-deleted HeV or NiV G protein.

The following oligonucleotide primers were synthesized for amplification of HeV G. sHGS: 5'-GTCGACCACCATGCAAAATTACACCAGAACGACTGATAAT-3' (SEQ ID NO: 5).

sHGAS: 5'-GTTTAAACGTCGACCAATCAACTCTCTGAACATTG
GGCAGGTATC-3'. (SEQ ID NO: 6).

The following oligonucleotide primers were synthesized for amplification of NiV G. sNGS: 5'-CTCGAGCACCATGCAAAATTACACAAGATCAACAGACAA-3' (SEQ ID NO: 7). 
sNGAS: 5'-CTCGAGTAGCAGCCGGATCAAGCTTATGTACATT
GCTCTGGTATC-3'. (SEQ ID NO: 8).
 All PCR reactions were done using Accupol DNA polymerase (PGS Scientifics Corp) with the following settings: 94°C for 5 minutes initially and then 94°C for 1 minute, 56°C for 2 minutes, 72°C for 4 minutes; 25 cycles. These primers generated a PCR product for the sHeV G ORF flanked by *Sal 1* sites and the sNiV G ORF flanked by *Xho* 1 sites. PCR products were gel purified (Qiagen). After gel purification, sHeV G and sNiV G were subcloned into a TOPO vector (Invitrogen).

PSectag2B (Invitrogen) was purchased and modified to contain a S-peptide tag or a myc-epitope tag. Overlapping oligonucleotides were synthesized that encoded the sequence for the S-peptide and digested *Kpn* 1 and EcoR1 overhangs.

SPEPS: 5'-CAAGGAGACCGCTGCTGCTAAGTTCGAACGCCAGCACATGGATT
CT-3' (SEQ ID NO: 9). SPEPAS: 5'AATTAGAATCCATGTGCTGGCGTTCGAACTT
AGCAGCAGCGGTCTCCTTGGTAC-3'. (SEQ ID NO: 10).

Overlapping oligonucleotides were synthesized that encoded the sequence for the myc-epitope tag and digested Kpn *1* and EcoR1 overhangs.
MTS: 5'-CGAACAAAAGCTCATCTCAGAAGAGGATCTG-3' (SEQ ID NO: 11). MTAS 5'-AATTCAGATCCTCTTCTGAGATGAGCTTTTGTTCGGTAC-3' (SEQ ID NO: 12).

64 pmol SPEPS and 64 pmol SPEPAS were mixed and heated to 65°C for 5 minutes and cooled slowly to 50°C. 64 pmol MTS and 64 pmol MTAS were mixed and heated to 65°C for 5 minutes and cooled slowly to 50°C. The two mixtures were diluted and cloned into *Kpn1-EcoR1* digested pSecTag2B to generate S-peptide modified pSecTag2B or myc-epitope modified pSecTag2B. All constructs were initially screened by restriction digest and further verified by sequencing.

The TOPO sG construct was digested with Sal 1 gel purified (Qiagen) and subcloned in frame into the *Xho* 1 site of the S-peptide modified pSecTag2B or myc-epitope modified pSecTag2B. All constructs were initially screened by restriction digest and further verified by sequencing.

The Igκ leader-S-peptide-s HeVG (sG_{S-tag}) and the Igκ leader-myc tag-sHeVG (sG_{myc-tag}) constructs were then subcloned into the vaccinia shuttle vector pMCO2. Oligonucleotide SEQS: 5'-TCGACCCACCATGGAGACAGACACACTCCTGCTA-3' (SEQ ID NO: 13) was synthesized and used in combination with oligonucleotide sHGAS to amplify by PCR the sG_{S-tag} and sG_{myc-tag}. All PCR reactions were done using Accupol DNA polymerase (PGS Scientifics Corp.) with the following settings: 94°C for 5 minutes initially and then 94°C for 1 minute, 56°C for 2 minutes, 72°C for 4 minutes; 25 cycles. These primers generated PCR products flanked by *Sal 1* sites. PCR products were gel purified (Qiagen). After gel purification, sG_{S-tag} and sG_{myc-tag} were subcloned into a TOPO vector (Invitrogen). sG S-tag and sG myc-tag were digested with *Sal 1* and subcloned into the *Sal 1* site of pMCO2. All constructs were initially screened by restriction digest and further verified by sequencing. A codon optimized nucleotide sequence was subsequently generated to facilitate production in euckaryotic cell lines which is depicted in SEQ ID NO: 16.

### Example 2: Protein Production of Soluble G Protein using Vaccinia

For protein production the genetic constructs containing the codon optimized sequences were used to generate recombinant poxvirus vectors (vaccinia virus, strain WR). Recombinant poxvirus was then obtained using standard techniques employing tk-selection and GUS staining. Briefly, CV-1 cells were transfected with either pMCO2 sHeV G fusion or pMCO2 sNiV G fusion using a calcium phosphate transfection kit (Promega). These monolayers were then infected with Western Reserve (WR) wild-type strain of vaccinia virus at a multiplicity of infection (MOI) of 0.05 PFU/cell. After 2 days the cell pellets were collected as crude recombinant virus stocks. TK⁻ cells were infected with the recombinant crude stocks in the presence of 25 µg/ml 5-Bromo-2'-deoxyuridine (BrdU) (Calbiochem). After 2 hours the virus was replaced with an EMEM-10 overlay containing 1% low melting point (LMP) agarose (Life Technologies) and 25 µg/ml BrdU. After 2 days of incubation an additional EMEM-10 overlay containing 1% LMP agarose, 25 µg/ml BrdU, and 0.2 mg/ml 5-Bromo-4-chloro-3-indolyl-β-D-glucuronic acid (X-GLUC) (Clontech) was added. Within 24-48 hours blue plaques were evident, picked and subject to two more rounds of double selection plaque purification. The recombinant vaccinia viruses vKB16 (sHeV G fusion) and vKB22 (sNiV G fusion) were then amplified and purified by standard methods. Briefly, recombinant vaccinia viruses are purified by plaque purification, cell-culture amplification, sucrose cushion pelleting in an ultracentrifuge and titration by plaque assay. Expression of sHeV G was verified in cell lysates and culture supernatants.

### Example 3: Protein Production of Soluble G Protein using 293F Cells

Genetic constructs containing the codon optimized sequences were used to transform 293F cells (Invitrogen) to produce a stable cell line which expresses HeV soluble G glycoprotein. CHO-S cells (Invitrogen) may also be used for transformation and expression of HeV soluble G glycoprotein. Transformed cells are plated on 162 cm² tissue culture flask with 35 ml DMEM-10. Cells were allowed to adhere and grow at 37°C with 5-8% CO₂ for several days. When cells were confluent, they were split into multiple flasks with DMEM-10 with 150 µg/ml Hygromycin B (30 ml per flask). When the cells are 70-80% confluent, they were washed twice with 30 ml PBS, then 20 ml of 293 SFM II (invitrogen) was added and the cells were incubated at 37°C with 5-8% CO₂ overnight. On the next day, cells were transferred into Erlenmeyer flasks with 200 ml SFM II media. Cells were allowed to grow at 37°C with 5-8% CO₂ at 125 rpm for 5-6 days until cells started to die. At that time, the supernatant is collected.

Media from each Erlenmeyer flask is centrifuged at 3,500 rpm for 30 minutes. The supernatant was then transferred into 250 ml centrifuge bottles and spun at 10,000 rpm for one hour. The resulting supernatant is collected and protease inhibitor is added according to manufacturer's recommendation along with Triton X-100 to final concentration of 0.1%. The supernatant is then filtered through a 0.2 µm low protein binding filter membrane.

HeVsG is purified through use of an S-protein agarose affinity column. A 20 ml bed volume of S-protein agarose (Novagen) is loaded into a XK 26 column (GE Healthcare). The column is washed with 10× bed volumes of Bind/Wash buffer (0.15 M NaCl, 20 mM Tris-HCl, pH 7.5 and 0.1% Triton X-100). The prepared supernatant of HeV sG is applied to the column to maintain a flow rate of 3 ml/min. The column is washed with 10× bed volumes (200 ml) of Bind/Wash buffer I followed by 6× bed volumes (120 ml) of wash buffer 1× Wash Buffer (0.15 M NaCl, and 20 mM Tris-HCl, pH 7.5).

The pump is then stopped and the Wash Buffer is allowed to drain until it reaches the surface of the beads when 30 ml of Elution Buffer (0.2 M Citric Acid, pH 2) is added. The first 10 ml of flow through (this should still be the wash buffer) is collected and then the elution buffer is incubated with the beads for 10 minutes. Next, 15 ml of the eluate is collected into a 50 mL sterile conical centrifuge tube containing 25 ml of neutralization buffer (1 M Tris, pH 8). The pH is adjusted to neutral and the elution and incubation is repeated three times. All of the neutralized eluate is combined and concentrated to about 4 ml. The collected HeV sG (4 ml) is purified through a 0.2 µm low protein binding filter membrane (Acrodisc 13 mm Syringe Filter with 0.2 µm HT Tuffryn Membrane.

Gel Filtration can be utilized to further purify the HeV sG. After quality control analysis and confirmation of purity and oligomeric state, aliquot HeV sG pooled fractions of tetramer+dimer, dimer and monomer are stored at -80°C.

### Example 4: Preparation of Vaccine Formulation

A schematic summarizing the preparation of ISC is set forth in Figure 3 and is further described below.

Step 1: A solution of 90 g/L decanoyl-n-methylglucamide (Mega-10 detergent) is prepared in Water For Injection-(WFI). The solution is heated to ensure total dissolution of Mega 10 then it is either used immediately in Step 2 or filter sterilized.

Step 2: A solution containing 25 g/L cholesterol and 25 g/L dipalmitoyl phosphatidyl choline (DPPC) is prepared by dissolving these components in the stock solution of Mega 10 detergent. The solution is heated to dissolve all components then either used immediately in Step 3 or filter sterilized.

Step 3: Buffered Isotonic Saline, 10 mM phosphate buffer, pH 6.2 ± 1 (BIS) is prepared with WFI and sterile filtered if not used immediately.

Step 4: Quil A is prepared in BIS to final concentration of 100 g/L and sterile filtered if not used immediately.

Step 5: ISC is formulated in an agitated temperature controlled vessel (22-37°C) by sequential addition of pre-heated BIS, cholesterol/DPPC in Mega-10 solution (160 ml/L), and Quil A solution (200 ml/L). The reaction is brought to target volume by addition of BIS.

Step 6: The entire formulation is equilibrated to the required temperature (Target 27°C with acceptable operating range 22-37°C) then incubated for 15 minutes with agitation to facilitate ISC formation. The ISC solution is either processed further in Step 7 or sterile filtered for intermediate storage.

Step 7: The ISC reaction mixture is washed by dialysis (Membrane: Hydrosart 30 kDa (Sartorius AG Goettingen)) for a minimum of 20 volume exchanges against BIS under temperature control (Target 27°C with acceptable operating range 21-37°C) to remove uncomplexed components.

Step 8: Dialyzed ISC is concentrated approximately 2-fold by ultra-filtration using the same membrane as that used for dialysis. The filtration system is rinsed with BIS to restore ISC to original volume.

Step 9: ISC is transferred to a sterile storage container via sterile filtration through a 0.22 µm cellulose acetate filter.

Step 10: ISC adjuvant is stored at 2-8°C until released for use in vaccine formulation.

The immunostimulatory composition (250 µg/ml) is then combined with appropriate amounts of soluble HeV G glycoprotein (e.g. 5, 50, 100 µg/ml) and adjusted to volume in BIS.

### Example 5: First Clinical Experiment in Horses

Test vaccine 1: Recombinant Hendra virus soluble glycoprotein (sG) at 100 µg/dose adjuvanted with 250 µg of immune stimulating complex; volume is adjusted to 1 ml/dose with saline solution.

Test vaccine 2: Recombinant Hendra virus soluble glycoprotein (sG) at 50 µg/dose adjuvanted with 250 µg of immune stimulating complex; volume is adjusted to 1 ml/dose with saline solution.

Test vaccine 3: Recombinant Hendra virus soluble glycoprotein (sG) at 5 µg/dose adjuvanted with 250 µg of immune stimulating complex; volume is adjusted to 1 ml/dose with saline solution.

Serological and challenge protection data from horses has been collected from two lots of horses given the vaccines containing the higher levels of antigen (50 µg/dose and 100 µg/dose).

Serology: Two horses were each immunized with two vaccine doses (100 µg sG with ISC) 21 days apart. Post-priming and pre-challenge serology confirmed vaccine-induced seroconversion to HeV (Table 1). Pre-challenge virus neutralizing antibody levels were comparable to those which had been found to be protective in cats exposed to an otherwise lethal dose of the closely related Nipah virus. The horse receiving adjuvant only (negative control) did not develop antibody to HeV prior to viral challenge.

**Table 1**

| Horse No. | Baseline titre | Post-prime titre | Pre-challenge titre |
|---|---|---|---|
| V1 | <2 | 32 | 1024 |
| V2 | <2 | 32 | 512 |
| V3 (Control) | <2 | <2 | <2 |

Accordingly, each horse was exposed to live HeV in a BSL4 containment facility 27 days after receiving the booster immunization. Virus was administered intranasally (1 × 10 ⁶TCID₅₀) and orally (1 × 10⁶ TCID₅₀). At the time of challenge and for the period of observation thereafter, the identity of the control horse was not known by staff involved in this part of the work.

Clinical observations for V1: This horse remained clinically well during the period of observation following exposure to HeV apart from a localized infection of the entry site of the indwelling jugular catheter noted on day 8 post-challenge. This was not associated with any constitutional signs of disease. The horse was electively euthanized on day 9 after viral challenge. Abnormalities at gross post mortem examination were confined to a 10 cm mesenteric lipoma (incidental finding) and mild dilation of lymphatic vessels at the ventral tip of the left cardiac lung lobe that was attributed to barbiturate. Initial screening of tissues has found no evidence of either lesions or HeV antigen in this horse.

Clinical observations for V2: This horse developed a mild transient nasal discharge on day 3, but then remained otherwise well until a temperature rise on day 6 associated with a localized inflammatory reaction at the site of her indwelling jugular catheter. The catheter was removed, but the lesion continued to enlarge and the horse was becoming quite irritable so on the following day (d 7) the mare was treated with long-acting penicillin. Both her temperature and her temperament had returned to normal on day 8 and she was electively euthanized. Abnormalities at gross post mortem examination were confined to mild dilation of lymphatic vessels at the ventral tip of the right cardiac lung lobe that was attributed to barbiturate. Initial screening of tissues has found no evidence of either lesions or HeV antigen in this horse; detailed examination is currently being completed.

Clinical observations for V3: This horse developed a mild transient nasal discharge on day 4, but then remained otherwise well until a temperature rise on day 6 without localizing signs. Her heart rate had also risen, and she had slight tenting of the skin consistent with mild dehydration and a tucked-up appearance. This constellation of signs is typical of acute HeV infection under our laboratory conditions. Her temperature and heart rate continued to increase over the ensuing 12 hours (Figure 1 and 2), she was slightly depressed, and so she was euthanized on humane grounds on day 7. At post mortem examination there was moderately severe dilation of lymphatic vessels on the cardiac lobes of the lung with involvement of the ventral 8-10 cm accompanied by pleural thickening and edema.

On histological examination there was pulmonary vasculitis with fibrinoid necrosis of vascular walls, edema of interlobular septa and focal necrotizing alveolitis. There was extensive deposition of HeV antigen in the endothelium and media of blood vessels in the lung; meninges; brain parenchyma; trigeminal ganglion; submandibular, bronchial, inguinal and renal lymph nodes; spleen; liver; heart; soft palate; adrenal gland; renal glomeruli; small and large intestines; ovary; pharynx and turbinates as well as germinal centers in the spleen and occasional cardiac myocytes. Spinal cord, guttural pouch, bladder, and olfactory pole of the brain were negative. The histology and immunohistology was consistent with peracute HeV infection.

Molecular analysis of clinical samples. There was no evidence for shedding of HeV in any biological sample collected from immunized horses V1 and V2 throughout the period of clinical observation. Specifically, no genome was recovered from either deep nasal swabs or from blood on any day post-exposure.

In contrast, in the non-immunized horse V3, viral genome was detected in nasal swabs from day 3 after challenge. Decreasing Ct values on successive sampling days is suggestive of viral replication in upper respiratory tract and is consistent with earlier observations from our laboratory following exposure of naïve horses to HeV Redlands 2008. The finding of viral genome in blood immediately prior to the onset of fever, and in all secretions thereafter, coinciding with the earliest recognition of other clinical signs such as depression is also consistent with earlier observations.

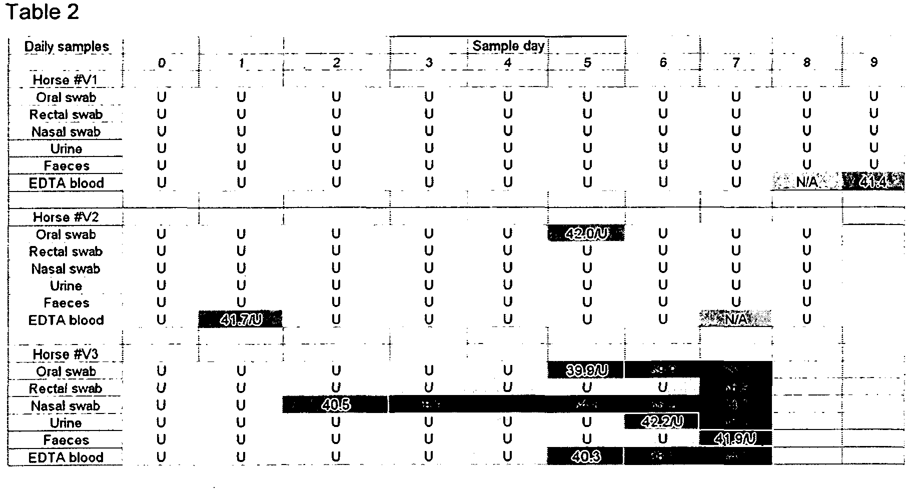

Post mortem samples. TaqMan PCR (HeV N-gene) confirmed replication of the challenge virus in V3 (Control) with dissemination of infection to multiple tissues (Table 3). Highest levels of replication appeared to be present in lung, spleen, kidney, myocardium, and lymphoid tissues associated with the upper and lower respiratory tracts as previously reported. There was no evidence of virus replication in tissues of immunized horses (V1 and V2).

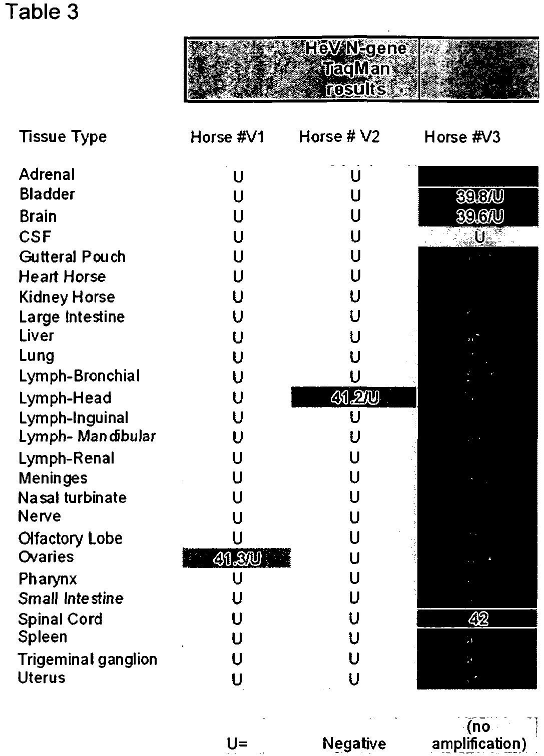

Post-challenge serology. immunized horses V1 and V2 did not have a boost in titre following HeV challenge (Table 4). This is consistent with no significant replication of the challenge virus in these animals. No antibody was detected in the control horse V3 at the time of euthanasia on post-challenge day 7. It was considered that there had been insufficient time between virus exposure and death of this animal for generation of detectable antibody, and is consistent with previous observations in our laboratory with HeV Redlands in the horse.

**Table 4**

| Horse No. | Baseline titre | Post-prime titre | Pre-challenge titre | Terminal titre |
|---|---|---|---|---|
| V1 | <2 | 32 | 1024 | 128, 128 (day 9) |
| V2 | <2 | 32 | 512 | 128, 256 (day 8) |
| V3 (Control) | <2 | <2 | <2 | <2, <2 (day 7) |

Two horses (V1 and V2) that were vaccinated with 100 µg sG + ISC adjuvant in a prime-boost regime seroconverted to HeV prior to HeV exposure. One horse (V3) that received ISC only remained seronegative to the challenge virus.

Following challenge with an otherwise lethal dose of HeV, immunized horses remained clinically well throughout the period of observation, which surpassed the time of onset of all experimentally induced cases of HeV in horses. The horse with no serological evidence of immunity (V3) was euthanized after developing clinical signs consistent with acute HeV. No boosting of antibody titre was detected following challenge in immunized horses, consistent with no replication of the challenge virus in these animals.

There was no evidence of viral shedding by immunized horses, as reflected by PCR negative test results on all daily clinical samples. In the non-immunized control, viral genome was detected in nasal swabs from day 3 after exposure to virus, in blood immediately prior to the onset of fever, and in all clinical samples from the time fever was established. This pattern of shedding is consistent with that found in naive horses exposed to HeV in an earlier study at this facility.

There was no evidence of HeV viral replication in any tissue of immunized horses collected at post mortem examination, following euthanasia during what would be expected to be the period of acute infection. In contrast, HeV genome and antigen were distributed throughout the tissues of the control horse in a pattern consistent with acute HeV infection, and vasculopathy typical of HeV infection was also identified.

### Example 6: Second Clinical Trial in Horses

Three horses were each immunized with two vaccine doses (50 µg sG with ISC) 21 days apart. Post-priming and pre-challenge serology confirmed vaccine-induced seroconversion to HeV (Table 5). Pre-challenge virus neutralizing antibody levels were comparable to those which had been found to be protective in cats exposed to an otherwise lethal dose of the closely related Nipah virus and to horses exposed to HeV in the first clinical trial described herein. A horse receiving adjuvant only did not develop antibody to HeV prior to viral challenge of immunized horses (data not displayed).

**Table 5**

| **Horse No.** | **Baseline titre** | **Post-prime titre** | **Pre-challenge titre** |
|---|---|---|---|
| V4 | <2 | 4 | 256/128 |
| V5 | <2 | 32 | 2048/>8192 |
| V6 | <2 | 4 | 512/1024 |

Accordingly, each immunized horse was exposed to live HeV in a BSL4 containment facility 27 days after receiving the booster immunization. Virus was administered intranasally (1 × 10⁶ TCID₅₀) and orally (1 × 10⁶ TCID₅₀). Four guinea pigs were employed in this study as pathogenicity controls with the expectation that at least one of these would succumb to HeV disease. Guinea pigs were exposed to 50,000 TCID₅₀ HeV by the intraperitoneal route.

Clinical observations for V4: This horse remained clinically well during the period of observation following exposure to HeV and temperature and heart rate remained within normal limits. The horse was electively euthanized on day 8 after viral challenge. No abnormalities were noted at gross post mortem examination. Initial screening of tissues has found no evidence of either lesions or HeV antigen in this horse; detailed examination is currently being completed.

Clinical observations for V5: This horse remained clinically well during the period of observation following exposure to HeV and temperature and heart rate remained within normal limits (Figure 2). The horse was electively euthanized on day 7 after viral challenge. No abnormalities were noted at gross post mortem examination. Initial screening of tissues has found no evidence of either lesions or HeV antigen in this horse; detailed examination is currently being completed.

Clinical observations for V6: This horse remained clinically well during the period of observation following exposure to HeV and temperature and heart rate remained within normal limits (Figure 2). The horse was electively euthanized on day 9 after viral challenge. No abnormalities were noted at gross post mortem examination. Initial screening of tissues has found no evidence of either lesions or HeV antigen in this horse; detailed examination is currently being completed.

Guinea pigs: One of 4 guinea pigs (No. 3) started to lose weight on day 3 after HeV challenge. Weight loss progressed until day 5 when the animal exhibited neurological signs (head tilt, tremor) and was euthanized. Abnormalities at post mortem examination were confined to edema of the retroperitoneal connective tissues.

On histological examination there was pulmonary vasculitis, vasculitis of peri-renal blood vessels, oophoritis, and non-suppurative encephalitis associated with deposition of HeV antigen. The histology and immunohistology were consistent with acute HeV infection and confirmed the pathogenicity of the challenge virus.

There was no evidence for shedding of HeV in any biological sample collected from V4, V5 or V6 throughout the period of clinical observation apart from a rectal swab from V6 on day 3 in which a Ct value (HeV N gene) of 36.2 was observed by TaqMan PCR in one of two replicate wells with the second well exhibiting no amplification (Table 6). Specifically, no genome was recovered from either deep nasal swabs or from blood on any day post-exposure.

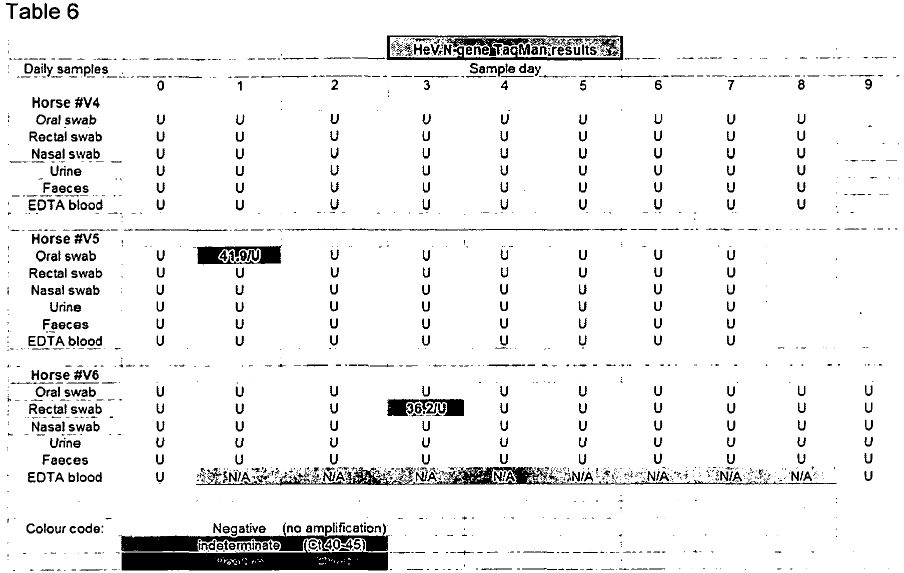

Post mortem samples. There was no evidence of virus replication in tissues of immunized horses V4, V5 or V6. In one guinea pig (No. 3), viral genome was detected in blood (Ct 34.2), brain, lung, and spleen on day 5 after challenge corroborating the clinical, histological and immunohistological findings of acute HeV infection in this animal (Table 7).

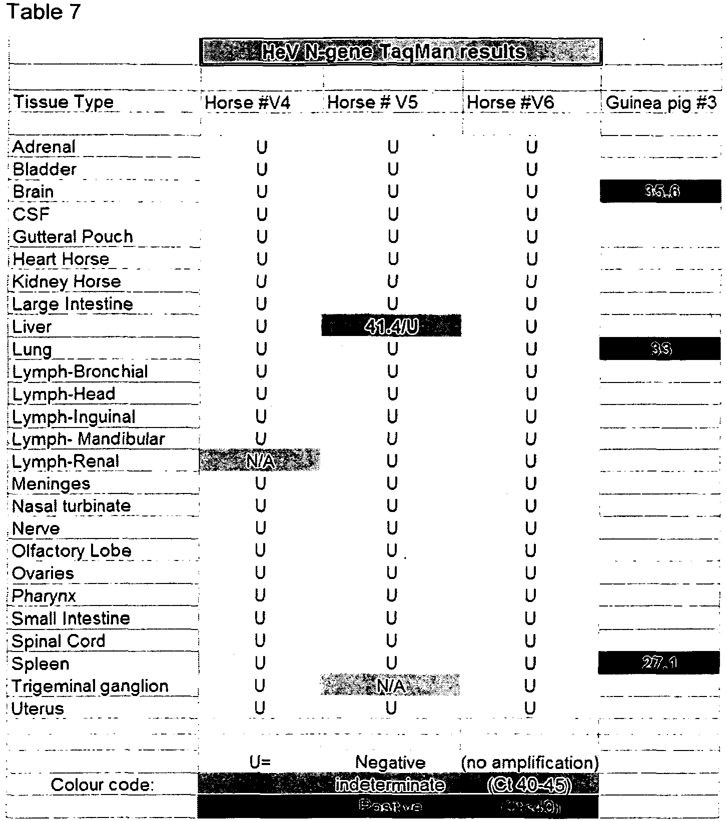

Post-challenge serology. Immunized horses V4, V5 and V6 did not have a boost in titre following HeV challenge (Table 8). This is consistent with no significant replication of the challenge virus in these animals.

**Table 8**

| Horse No. | Baseline titre | Post-prime titre | Pre-challenge titre | Terminal titre |
|---|---|---|---|---|
| V4 | <2 | 4 | 256/128 | 256/32 (day 8) |
| V5 | <2 | 32 | 2048/>8192 | 1024/512 (day 7) |
| V6 | <2 | 4 | 512/1024 | 128/256 (day 9) |

Three horses (V4, V5 and V6) that were vaccinated with 50 µg sG + ISC adjuvant in a prime-boost regime seroconverted to HeV prior to HeV exposure. One horse that received ISC only remained seronegative to the challenge virus.

Following challenge with an otherwise lethal dose of HeV, immunized horses remained clinically well throughout the period of observation, which surpassed the time of onset of all experimentally induced cases of HeV in horses. One guinea pig used as a pathogenicity control was euthanized after developing clinical signs consistent with acute HeV. No boosting of antibody titre was detected following challenge in immunized horses, consistent with no replication of the challenge virus in these animals.

There was no evidence of viral shedding by immunized horses, as reflected by PCR negative test results on all daily clinical samples apart from one replicate from a rectal swab from V6 on day 3. This test is being repeated; should a similar result be observed one explanation is that this represents a low level of residual inoculum. In one non-immunized guinea pig, viral genome was detected in major organs and blood on day 5 after exposure to virus.

There was no evidence of HeV viral replication in any tissue of immunized horses collected at post mortem examination, following euthanasia during what would be expected to be the period of acute infection. In contrast, HeV genome and antigen were distributed throughout the tissues of a susceptible guinea pig in a pattern consistent with acute HeV infection, and vasculopathy typical of HeV infection was also identified in this animal.

### Example 7: Clinical Trial in Primates for Nipah Virus

Statistics. Conducting animal studies, in particular non-human primate studies, in biosafety level 4 (BSL-4) severely restricts the number of animal subjects, the volume of biological samples that can be obtained and the ability to repeat assays independently and thus limit statistical analysis. Consequently, data are presented as the mean or median calculated from replicate samples, not replicate assays, and error bars represent the standard deviation across replicates.

Viruses. NiV-Malaysia (GenBank Accession No. AF212302) was obtained from the Special Pathogens Branch of the Centers for Disease Control and Prevention, Atlanta, Georgia. NiV was propagated and titered on Vero cells as described for HeV in Rockx et al. (2010) J. Virol. 84, 9831.

Vaccine formulation. Three vaccine formulations of sGHeV were employed (10 µg, 50 µg or 100 µg). Production and purification of sGHeV was done as previously described in Pallister (2011) Vaccine 29, 5623. Each vaccine formulation also contained Allhydrogel^{™} (Accurate Chemical & Scientific Corporation) and CpG oligodeoxynucleotide (ODN) 2006 (Invivogen) containing a fully phosphorothioate backbone. Vaccine doses containing fixed amount of ODN 2006, varying amounts of sGHeV and aluminum ion (at a weight ratio of 1:25) were formulated as follows: 100 µg dose: 100 µg sGHeV, 2.5 mg aluminum ion and 150 µg of ODN 2006; 50 µg dose: 50 µg sGHeV, 1.25 mg aluminum ion and 150 µg of ODN 2006; and 10 µg dose: 5 µg sGHeV, 250 µg aluminum ion and 150 µg of ODN 2006. For all doses, Alhydrogel^{™} and sGHeV were mixed first before ODN 2006 was added. Each vaccine dose was adjusted to 1 ml with PBS and mixtures were incubated on a rotating wheel at room temperature for at least two to three hours prior to injection. Each subject received the same 1 ml dose for prime and boost and all vaccine doses were given via intramuscular injection.

Animals. Ten young adult African Green Monkeys (AGM) (Chlorocebus aethiops), weighing 4-6 kg (Three Springs Scientific Inc.) were caged individually. Subjects were anesthetized by intramuscular injection of ketamine (10-15 mg/kg) and vaccinated with sGHeV on day -42 (prime) and day -21 (boost). Three subjects received two 10 µg doses (AGM 16, AGM 17, AGM 18), three subjects received two 50 µg doses (AGM 13, AGM 14, AGM 15), three animals received two 100 µg doses (AGM 10, AGM 11, AGM 12) and one subject (AGM 9) received adjuvant-alone. On day 0, subjects were anesthetized and inoculated intratracheally with 1 × 10⁵ TCID₅₀ (median tissue culture infectious dose) of NiV in 4 ml of Dulbecco's minimal essential medium (DMEM) (Sigma-Aldrich). Subjects were anesthetized for clinical examinations including temperature, respiration rate, chest radiographs, blood draw and swabs of nasal, oral and rectal mucosa on days 0, 3, 5, 7, 10, 14, 21 and 28 post-infection (p.i.). The control subject (AGM 9) had to be euthanized according to approved humane end points on day 10 post-infection. All other subjects survived until the end of the study and were euthanized on day 28 post-infection. Upon necropsy, various tissues were collected for virology and histopathology. Tissues sampled include: conjunctiva, tonsil, oro/naso pharynx, nasal mucosa, trachea, right bronchus, left bronchus, right lung upper lobe, right lung middle lobe, right lung lower lobe, light lung upper lobe, light lung middle lobe, light lung lower lobe, bronchial lymph node (LN), heart, liver, spleen, kidney, adrenal gland, pancreas, jejunum, colon transversum, brain (frontal), brain (cerebellum), brain stem, cervical spinal cord, pituitary gland, mandibular LN, salivary LN, inguinal LN, axillary LN, mesenteric LN, urinary bladder, testes or ovaries, femoral bone marrow. Vaccination was done under BSL-2 containment. A timeline of the vaccination schedule, challenge and biological specimen collection days is shown in Figure 4.

Vaccination and NiV challenge. Previously, we have demonstrated that intratracheal inoculation of AGMs with 10⁵ TCID₅₀ (median tissue culture infectious dose) of NiV caused a uniformly lethal outcome (Rockx et al. (2010) J. Virol. 84, 9831). Rapidly progressive clinical illness was noted in these studies; clinical signs included severe depression, respiratory disease leading to acute respiratory distress, severe neurological disease and severely reduced mobility; and time to reach approved humane endpoint criteria for euthanasia ranged from 7 to 12 days. Here we sought to determine if vaccination with sGHeV could prevent NiV infection and disease in AGMs. Doses of 10, 50 or 100 µg sGHeV were mixed with alum and CpG moieties as described in the Methods. Each vaccine formulation was administered subcutaneously to three subjects on day 0 (prime) and again on day 21 (boost) and one control subject (AGM 9) received an adjuvant alone prime and boost on the same days. On day 42, all subjects were inoculated intratracheally with 10⁵ TCID₅₀ NiV. The control subject (AGM 9) showed loss of appetite, severe sustained behavior changes (depression, decreased activity, hunched posture), decreases in platelet number and a gradual increase in respiratory rate at end-stage disease. Subsequently, AGM 9 developed acute respiratory distress and had to be euthanized according to approved humane end points on day 10 post-infection. In contrast, none of the vaccinated subjects had clinical disease and all survived until the end of the study. A Kaplan-Meier survival graph is shown in Figure 5.

NiV-mediated disease in the control subject. Gross pathological changes in the control subject were consistent with those found previously in NiV-infected AGMs (Geisbert et al. (2010) PLoS One 5, e10690). Splenomegaly and congestion of blood vessels on surface of brain were present and all lung lobes were wet and heavy. NiV RNA and infectious virus were not recovered from AGM 9 blood samples and there was no evidence of viremia. AGM 9 had significant levels of NiV-specific IgM and detectable NiV-specific IgG and IgA. Further analysis of tissue samples revealed an extensive NiV tissue tropism similar to the wide-spread NiV infection seen previously in AGMs (Geisbert et al. (2010) PLoS One 5, e10690). AGM 9 had NiV RNA in the majority of tissues as indicated and infectious virus was recovered from numerous tissues. Significant lesions included interstitial pneumonia, subacute encephalitis and necrosis and hemorrhage of the splenic white pulp. Alveolar spaces were filled by edema fluid, fibrin, karyorrhectic and cellular debris, and alveolar macrophages. Multifocal encephalitis was characterized by expansion of Virchow-Robins space by moderate numbers of lymphocytes and fewer neutrophils. Smaller numbers of these inflammatory cells extended into the adjacent parenchyma. Numerous neurons were swollen and vacuolated (degeneration) or were fragmented with karyolysis (necrosis). Multifocal germinal centers of follicles in splenic white pulp were effaced by hemorrhage and fibrin, as well as small numbers of neutrophils and cellular and karyorrhectic debris. These findings were consistent with necrosis and loss of the germinal centers in the spleen. Extensive amounts of viral antigen were present in the brainstem highlight the extensive damage NiV causes in the central nervous system.

Protection of sGHeV-vaccinated subjects. All biological specimens, including all blood samples collected following challenge and all tissues collected upon necropsy, were negative for NiV RNA and infectious virus was not isolated from any specimen. Upon closer examination of tissue sections from vaccinated subjects, tissue architecture appeared normal and NiV antigen was not detected in any tissue using immunohistochemical techniques. To further dissect the vaccine-elicited mechanisms of protection, serum and mucosal sGNiV- and sGHeV-specific IgM, IgG and IgA as well as NiV and HeV serum neutralization titers were measured in vaccinated animals. As demonstrated in Figure 6, seven days prior to challenge, subjects receiving the lowest sGHeV dose had detectable antigen-specific serum IgM and the highest level of sGHeV-specific serum IgG. Subjects given 50 µg sGHeV also had detectable levels of serum IgM and their highest levels of serum IgG seven days prior to challenge. High dose subjects had no detectable serum IgM and serum IgG levels were significantly less on day -7 as compared to the other two groups. By the day of NiV challenge, serum IgG levels in the high dose subjects had increased and all vaccinated subjects had similar IgG levels. Serum IgM levels did not change in any subject following NiV challenge. Serum IgG levels decreased in the medium dose subjects the day of NiV challenge and IgG levels decreased in low dose subjects just after NiV challenge. Interestingly, IgG levels increased in both of these groups by day 3 and day 5 p.i. but never surpassed the IgG levels present seven days prior to challenge and in both groups titer decreased significantly by day 28 p.i..

Conversely, serum IgG levels in the high dose group remained high and were at their highest of day 28 p.i.. Antigen-specific serum IgA was detectable in all subjects following vaccination; however, levels were very low and pre- and post-challenge levels did not appear to be significantly different (Figure 6). A minimal increase in mucosal antigen-specific IgA was detected in nasal swabs from low dose subjects on day 14 p.i., however, the levels were so low these mucosal antibodies likely played no role in preventing the spread of NiV following challenge. Results from serum neutralization tests (SNTs) are shown in Table 9. For all vaccinated subjects, HeV-specific neutralization titer remained the same or decreased by day 28 p.i. and NiV-specific neutralization titer did not change significantly by day 7 p.i., even in subjects that had the lowest titer prior to challenge. One low dose and one high dose subject had a log increase in NiV SNT titer by day 14 p.i. and one medium dose subject had a log increase in NiV SNT titer by day 21 p.i. For all other vaccinated animals, changes in SNT titer were either inconsistent (titer would increase and then decrease) or insignificant (titer increased by 3-4 fold but not more than a log). Finally, seroconversion to the NiV fusion (F) envelope glycoprotein was measured in vaccinated subjects following NiV challenge. Minimal levels of serum anti-NiV F IgM were detected in the low and medium dose subjects on day 10 and day 21 p.i., respectively, and these low M.F.I. values suggest a weak primary antibody response following NiV challenge. Serum anti-NiV-F IgM was not detected in the high dose subjects suggesting these animals had little to no circulating virus following challenge.

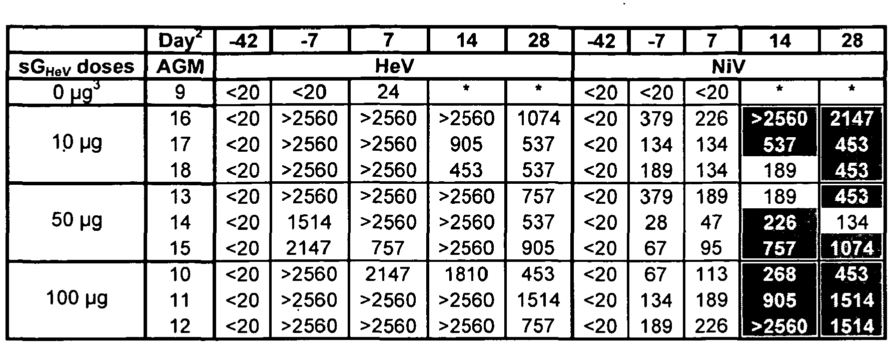

### Example 8: Clinical Trial in Primates for Hendra Virus

A second clinical trial was conducted in AGM to assess vaccination and challenge with Hendra virus. The same formulation as set forth in Example 7 was utilized as a vaccine but was also compared to another group that received sGHeV with Alhydrogel^{™} alone as an adjuvant (no ODN 2006 was present). Animals were vaccinated day -21, boosted on day 0, and challenged on day 21. Unless otherwise indicated, all conditions were the same as those on Example 7. An experimental summary is below:

| **Group** | **Treatment** | **N** | **Dosing regimen** |
|---|---|---|---|
| **A** | 100 µg/dose Hendra sG vaccine + adjuvants (150 µg CpG ODN 2006 + 119 µl Alhydrogel) | 4 | Prime + 1 boost separated by 3 weeks |
| **B** | 100 µg/dose Hendra sG vaccine + adjuvant (250 µl Alhydrogel) | 4 | Prime + 1 boost separated by 3 weeks |
| **C** | Adjuvant only (150 µg CpG ODN 2006) | 1 | Prime + 1 boost separated by 3 weeks |
| **D** | Adjuvants only (250 µl Alhydrogel ) | 1 | Same schedule as Groups A-B |
| **Total** | | **10** | |

Result: All animals (n=4) in both groups (A and B) survived Hendra virus challenge after being inoculated intratracheally with 10⁵ TCID₅₀ Hendra virus. Control subjects died on day 8. No clinical illness was observed in any of the vaccinated subjects and they remained healthy and well until study endpoint.

## Claims

1. A vaccine comprising:
a. a soluble fragment of the Nipah virus G glycoprotein, said fragment consisting of amino acids 71 to 602 of SEQ ID NO: 4 or a sequence at least 95% identical thereto;
b. an immunostimulatory complex (ISC), said ISC comprising a saponin; a phospholipid selected from the group consisting of phosphatidyl choline (PC), dipalmitoyl phosphatidyl choline (DPPC), phosphatidic acid (phosphatidate) (PA), phosphatidylethanolamine (PE), phosphatidylserine (PS), phosphatidylinositol (PI), phosphatidylinositol phosphate (PIP), phosphatidylinositol bisphosphate (PIP2), phosphatidylinositol triphosphate (PIP3), phosphorylcholine (SPH), ceramide phosphorylethanolamine (Cer-PE) and ceramide phosphorylglycerol; and cholesterol; and
c. one or more excipients;
for use in prevention of an infection caused by Hendra and/or Nipah virus in a horse or a pig, wherein said vaccine is administered to said horse or said pig in a first dose and a second dose, wherein each of said doses comprises 50-100 µg of said soluble fragment of the Nipah virus G glycoprotein.

2. The vaccine for use according to claim 1, wherein each dose comprises 100 µg of said soluble fragment of the Nipah virus G glycoprotein.

3. The vaccine for use according to claim 1 or claim 2, wherein the soluble Nipah virus G glycoprotein is present in dimer form.

4. The vaccine for use according to claim 1 or claim 2, wherein the soluble Nipah virus G glycoprotein is present in tetramer form.

5. The vaccine for use according to any one of claims 1-4, wherein each dose comprises 250 µg of ISC.

6. The vaccine for use according to any one of claims 1-5, wherein the saponin is Quil A and the phospholipid is DPPC.

7. The vaccine for use according to claim 6, wherein the ratio of Quil A: DPPC: Cholesterol in the composition is 5:1:1 by weight.

8. The vaccine for use according to any one of claims 1-7, wherein the second dose is administered 21-28 days after the first dose.

## Patentansprüche

1. Impfstoff, umfassend:
a. ein lösliches Fragment des Nipahvirus-G-Glycoproteins, wobei das Fragment aus den Aminosäuren 71 bis 602 von SEQ ID NO: 4 oder einer Sequenz, die mindestens 95% identisch dazu ist, besteht;
b. einen immunstimulatorischen Komplex (ISC), wobei der ISC ein Saponin; ein Phospholipid ausgewählt aus der Gruppe, bestehend aus Phosphatidylcholin (PC), Dipalmitoylphosphatidylcholin (DPPC), Phosphatidsäure (Phosphatidat) (PA), Phosphatidylethanolamin (PE), Phosphatidylserin (PS), Phosphatidylinositol (PI) Phosphatidylinositolphosphat (PIP), Phosphatidylinositolbisphosphat (PIP2), Phosphatidylinositoltriphosphat (PIP3), Phosphorylcholin (SPH), Ceramidphosphorylethanolamin (Cer-PE) und Ceramidphosphorylglycerin; und Cholesterin umfasst; und
c. einen oder mehrere Exzipienten;
zur Verwendung bei der Prävention einer Infektion, die durch Hendra- und/oder Nipahvirus verursacht wird, in einem Pferd oder einem Schwein, wobei der Impfstoff dem Pferd oder dem Schwein in einer ersten Dosis und einer zweiten Dosis verabreicht wird, wobei die Dosen jeweils 50 bis 100 µg des löslichen Fragments des Nipahvirus-G-Glycoproteins umfassen.

2. Impfstoff zur Verwendung nach Anspruch 1, wobei jede Dosis 100 µg lösliches Fragment des Nipahvirus-G-Glycoproteins umfasst.

3. Impfstoff zur Verwendung nach Anspruch 1 oder Anspruch 2, wobei das lösliche Nipahvirus-G-Glycoprotein in Dimerform vorliegt.

4. Impfstoff zur Verwendung nach Anspruch 1 oder Anspruch 2, wobei das lösliche Nipahvirus-G-Glycoprotein in Tetramerform vorliegt.

5. Impfstoff zur Verwendung nach einem der Ansprüche 1 bis 4, wobei jede Dosis 250 µg ISC umfasst.

6. Impfstoff zur Verwendung nach einem der Ansprüche 1 bis 5, wobei das Saponin Quil A ist und das Phospholipid DPPC ist.

7. Impfstoff zur Verwendung nach Anspruch 6, wobei das Verhältnis Quil A: DPPC: Cholesterin in der Zusammensetzung 5:1:1, bezogen auf das Gewicht, beträgt.

8. Impfstoff zur Verwendung nach einem der Ansprüche 1 bis 7, wobei die zweite Dosis 21 bis 28 Tage nach der ersten Dosis verabreicht wird.

## Revendications

1. Vaccin comprenant :
a. un fragment soluble de la glycoprotéine G du virus Nipah, ledit fragment étant constitué des acides aminés 71 à 602 de SEQ ID NO : 4 ou une séquence au moins 95 % identique à celle-ci ;
b. un complexe immunostimulant (ISC), ledit ISC comprenant une saponine ; un phospholipide choisi dans le groupe constitué de : la phosphatidylcholine (PC), la dipalmitoyl-phosphatidylcholine (DPPC), l'acide phosphatidique (phosphatidate) (PA), la phosphatidyléthanolamine (PE), la phosphatidylsérine (PS), le phosphatidylinositol (PI), le phosphate de phosphatidylinositol (PIP), le bisphosphate de phosphatidylinositol (PIP2), le triphosphate de phosphatidylinositol (PIP3), la phosphorylcholine (SPH), la céramide phosphoryléthanolamine (Cer-PE) et le céramide phosphorylglycérol ; et le cholestérol ; et
c. un ou plusieurs excipients ;
pour utilisation dans la prévention d'une infection causée par le virus Hendra et/ou Nipah chez un cheval ou un porc, ledit vaccin étant administré audit cheval ou audit porc dans une première dose et une deuxième dose, chacune desdites doses comprenant 50 à 100 µg dudit fragment soluble de la glycoprotéine G du virus Nipah.

2. Vaccin pour utilisation selon la revendication 1, dans lequel chaque dose comprend 100 µg dudit fragment soluble de la glycoprotéine G du virus Nipah.

3. Vaccin pour utilisation selon la revendication 1 ou la revendication 2, dans lequel la glycoprotéine G soluble du virus Nipah est présente sous forme de dimère.

4. Vaccin pour utilisation selon la revendication 1 ou la revendication 2, dans lequel la glycoprotéine G soluble du virus Nipah est présente sous forme de tétramère.

5. Vaccin pour utilisation selon l'une quelconque des revendications 1 à 4, dans lequel chaque dose comprend 250 µg d'ISC.

6. Vaccin pour utilisation selon l'une quelconque des revendications 1 à 5, dans lequel la saponine est Quil A et le phospholipide est DPPC.

7. Vaccin pour utilisation selon la revendication 6, dans lequel le rapport de Quil A : DPPC : cholestérol dans la composition est 5:1:1 en poids.

8. Vaccin pour utilisation selon l'une quelconque des revendications 1 à 7, la deuxième dose étant administrée 21 à 28 jours après la première dose.
